# EUROPEAN PATENT APPLICATION

(11) **EP 4 834 307 A1**
(43) Date of publication of application: **04.11.2026**
(21) Application number: 24913013.9
(22) Date of filing: 26.12.2024
(51) Int. Cl.: A61K 6/884

(54) **DENTAL COMPOSITION**

(30) Priority: 28.12.2023 JP 2023223465
(71) Applicant: Kuraray Noritake Dental Inc., Kurashiki-shi, Okayama 710-0801 (JP)
(72) Inventor: MURAYAMA, Ryota, Tainai-shi, Niigata 959-2653 (JP); MURAYAMA, Momoe, Tainai-shi, Niigata 959-2653 (JP); KAWANA, Mariko, Tainai-shi, Niigata 959-2653 (JP); MATSUURA, Ryo, Tainai-shi, Niigata 959-2653 (JP)
(74) Representative: D Young & Co LLP
(86) International application number: PCT/JP2024/046167
(87) International publication number: WO 2025/143127

(57) **Abstract**

The present invention provides a dental composition that exhibits excellent photocurability while having superior ambient light stability under high illuminance. The present invention relates to a dental composition comprising an ultraviolet absorber (A), a polymerizable monomer (B), and a photopolymerization initiator (C),
wherein the ultraviolet absorber (A) is a compound that has:
[I] a triazine skeleton;
[II] a solubility of 0.12 parts or more by mass relative to 100 parts by mass of 2-hydroxyethyl methacrylate; and
[III] a maximum absorption wavelength in a wavelength range of 310 nm or more and 400 nm or less as measured over a wavelength range of 250 nm to 500 nm for a solution prepared at a concentration of 3 × 10⁻⁵ mol/L by dissolving the ultraviolet absorber (A) in ethyl acetate serving as solvent, using a spectrophotometer with a quartz cell having an optical path length of 10 mm.

## Description

### TECHNICAL FIELD

The present invention relates to a dental composition. More specifically, the invention relates to a dental composition that exhibits excellent photocurability while having superior ambient light stability under high illuminance.

### BACKGROUND ART

Restorative filling materials such as filling composite resins and filling compomers, and crown restoration materials such as metal alloys, porcelain, and resin materials are typically used for the restoration of damaged tooth structures (enamel, dentin, and cementum) caused by caries and other dental defects.

As a rule, restorative filling materials and crown restoration materials (in this specification, these are also referred to collectively as "dental restoration materials") themselves do not possess adhesive properties to the tooth structure. Consequently, various adhesive systems with bonding agents have been used for the bonding of the tooth structure and dental restoration materials.

An example of adhesive systems that are in common use is the acid etching (total etching) adhesive system, whereby a surface of tooth structure is etched with an acid etchant such as a phosphoric acid aqueous solution, and a bonding material, or an adhesive, is applied to bond a dental restoration material to the tooth structure.

The self-etching adhesive system, on the other hand, is an example of adhesive systems that do not use acid etchants. In the past, the mainstream in such adhesive systems has been a two-step process in which a self-etching primer containing an acidic monomer, a hydrophilic monomer, and water is first applied to a surface of tooth structure, and a bonding material containing a crosslinkable monomer and a polymerization initiator is applied without rinsing the surface with water. Another type of self-etching adhesive system that has come to be commonly used in recent years is the single-step adhesive system, which uses a one-part dental adhesive (one-part dental bonding material) that serves as both a self-etching primer and a bonding material.

In these types of adhesive systems, the dental bonding material is usually cured under light after being applied to the site requiring restoration. For this reason, a photopolymerization initiator is employed in these adhesive systems.

For curing, the dental bonding material is exposed to light from a photoirradiator after it is applied to a cavity prepared by removing caries. Needless to say, this photoirradiation should sufficiently cure the bonding material layer.

However, in clinical settings, the bonding material should remain uncured under ambient light, such as fluorescent lighting or dental lamps in the treatment room of a dental clinic, during the interval from dispensing of the bonding material to its application to the cavity.

Against this backdrop, Patent Literatures 1 to 4 propose dental compositions.

### CITATION LIST

### Patent Literature

Patent Literature 1: JP2016-166138 A
Patent Literature 2: JP2006-11754 A
Patent Literature 3: JP2018-104367 A
Patent Literature 4: JP2004-231913 A

### SUMMARY OF INVENTION

### Technical Problem

In recent years, there has been a trend toward increasing brightness in dental lighting, particularly operating lights used to illuminate the dentist's working field and the oral cavity. This has created a need for a dental bonding material that can satisfy two opposing properties at a higher level: superior ambient light stability under high illuminance (resistance to curing) and excellent photocurability (curing upon exposure to light from a photoirradiator).

However, with respect to achieving a balance between ambient light stability under high illuminance and photocurability, there remain areas for improvement in the dental compositions according to Patent Literatures 1 to 4.

It is an object of the present invention to provide a dental composition that exhibits excellent photocurability while having superior ambient light stability under high illuminance.

### Solution to Problem

The present inventors conducted intensive studies to find a solution to the foregoing issue, and found that the problem can be solved by incorporating an ultraviolet absorber that has a triazine skeleton, a solubility of 0.12 parts or more by mass relative to 100 parts by mass of 2-hydroxyethyl methacrylate, and a maximum absorption wavelength in a wavelength range of 310 nm or more and 400 nm or less. Building upon this finding, the present inventors conducted additional examinations, ultimately resulting in the completion of the present invention.

Specifically, the present invention includes the following.
[1] A dental composition comprising an ultraviolet absorber (A), a polymerizable monomer (B), and a photopolymerization initiator (C),
   wherein the ultraviolet absorber (A) is a compound that has:
   [I] a triazine skeleton;
   [II] a solubility of 0.12 parts or more by mass relative to 100 parts by mass of 2-hydroxyethyl methacrylate; and
   [III] a maximum absorption wavelength in a wavelength range of 310 nm or more and 400 nm or less as measured over a wavelength range of 250 nm to 500 nm for a solution prepared at a concentration of 3 × 10⁻⁵ mol/L by dissolving the ultraviolet absorber (A) in ethyl acetate serving as solvent, using a spectrophotometer with a quartz cell having an optical path length of 10 mm.
[2] The dental composition according to [1], wherein the ultraviolet absorber (A) is a triazine-based ultraviolet absorber represented by the following general formula (1), 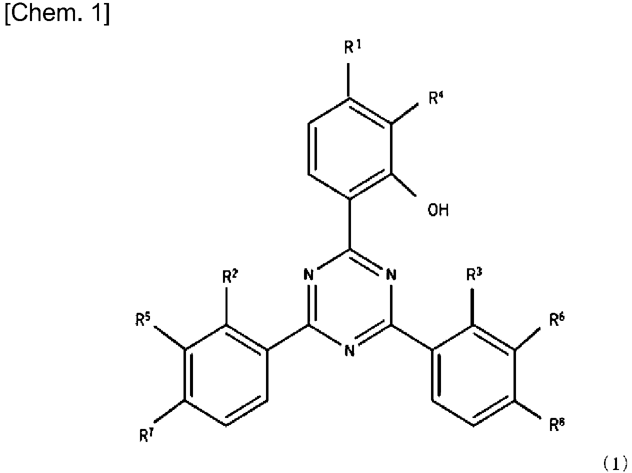 wherein R¹ represents an alkyl group or an alkoxy group, R² and R³ are the same or different from each other and each represent a hydrogen atom, a hydroxyl group, an alkyl group, an alkoxy group, or a halogen atom, R⁴, R⁵, and R⁶ are the same or different from each other and each represent a hydrogen atom or an alkyl group, and R⁷ and R⁸ are the same or different from each other and each represent a hydrogen atom, a hydroxyl group, an alkyl group, an alkoxy group, an aryl group, or a halogen atom, where the alkyl groups and alkoxy groups of R¹ to R⁸ may be optionally substituted.
[3] The dental composition according to [1] or [2], wherein the content of the ultraviolet absorber (A) is 85 parts or more by mass in total 100 parts by mass of ultraviolet absorbers.
[4] The dental composition according to [2] or [3], wherein R¹ in the ultraviolet absorber (A) is an alkyl group or alkoxy group having 1 to 15 carbon atoms.
[5] The dental composition according to [4], wherein R¹ in the ultraviolet absorber (A) is a branched alkyl group or alkoxy group having 3 to 15 carbon atoms.
[6] The dental composition according to [5], wherein R¹ in the ultraviolet absorber (A) is a 2-ethylhexyloxy group.
[7] The dental composition according to any one of [1] to [6], wherein the ultraviolet absorber (A) is bisethylhexyloxyphenol methoxyphenyl triazine and/or 2-[4,6-bis(biphenyl-4-yl)-1,3,5-triazin-2-yl]-5-[(2-ethylhexyl)oxy]phenol.
[8] The dental composition according to any one of [1] to [7], wherein the content of the ultraviolet absorber (A) is 0.01 parts or more by mass and 1 part or less by mass relative to total 100 parts by mass of the polymerizable monomer (B) contained in the dental composition.
[9] The dental composition according to any one of [1] to [8], wherein the mass ratio (A):(C) of the content of the ultraviolet absorber (A) and the content of the photopolymerization initiator (C) is 1:0.1 to 1:50.
[10] The dental composition according to any one of [1] to [9], wherein the photopolymerization initiator (C) is an α-diketone and/or a (bis)acylphosphine oxide.
[11] The dental composition according to [10], wherein the content of the photopolymerization initiator (C) is 0.01 parts or more by mass and 5 parts or less by mass in 100 parts by mass of the dental composition.
[12] The dental composition according to any one of [1] to [10], wherein the dental composition is a one-pack type dental composition.

### Advantageous Effects of Invention

According to the present invention, a dental composition can be provided that exhibits excellent photocurability while having superior ambient light stability under high illuminance.

### DESCRIPTION OF EMBODIMENTS

A dental composition of the present invention comprises an ultraviolet absorber (A), a polymerizable monomer (B), and a photopolymerization initiator (C),
wherein the ultraviolet absorber (A) is a compound that has:
[I] a triazine skeleton;
[II] a solubility of 0.12 parts or more by mass relative to 100 parts by mass of 2-hydroxyethyl methacrylate; and
[III] a maximum absorption wavelength in a wavelength range of 310 nm or more and 400 nm or less as measured over a wavelength range of 250 nm to 500 nm for a solution prepared at a concentration of 3 × 10⁻⁵ mol/L by dissolving the ultraviolet absorber (A) in ethyl acetate serving as solvent, using a spectrophotometer with a quartz cell having an optical path length of 10 mm.

As used herein, "(meth)acryl" is a collective term for methacryl and acryl.

In this specification, the upper limits and lower limits of numeric ranges (including, for example, the content of each component, values calculated from components, and various physical properties) can be appropriately combined.

### [Ultraviolet Absorber (A)]

The ultraviolet absorber (A) used in a dental composition of the present invention is a triazine-based ultraviolet absorber that has a maximum absorption wavelength in a wavelength range of 310 nm or more and 400 nm or less.

The method for measuring the maximum absorption wavelength is as described in the EXAMPLES below.

Triazine-based ultraviolet absorbers show stronger absorption in the ultraviolet region than other types of ultraviolet absorbers. It is presumed that those having a maximum absorption wavelength in a wavelength range of 310 nm or more and 400 nm or less are more stable under high-illuminance ambient light such as operating lights, and do not interfere with irradiation from a blue LED photoirradiator for curing, leading to superior photocurability.

It is also speculated that, because the ultraviolet absorber (A) according to the present invention is chosen for its particularly superior solubility in monomers, the ultraviolet absorber is dispersed throughout the dental composition, ensuring reliable ultraviolet absorption without excess or deficiency, leading to notably superior stability to ambient light under high illuminance.

In view of even superior ambient light stability under high illuminance and even superior photocurability, the ultraviolet absorber (A) is preferably a triazine-based ultraviolet absorber represented by the following general formula (1), 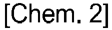 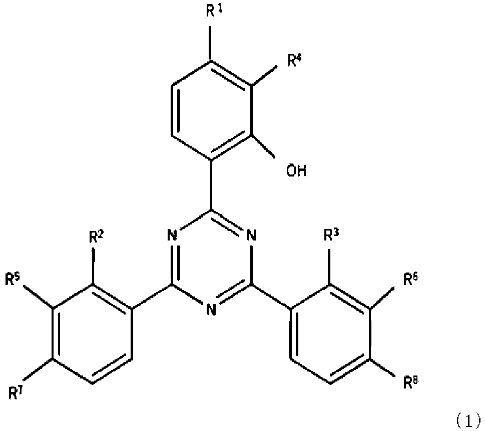 wherein R¹ represents an alkyl group or an alkoxy group, R² and R³ are the same or different from each other and each represent a hydrogen atom, a hydroxyl group, an alkyl group, an alkoxy group, or a halogen atom, R⁴, R⁵, and R⁶ are the same or different from each other and each represent a hydrogen atom or an alkyl group, and R⁷ and R⁸ are the same or different from each other and each represent a hydrogen atom, a hydroxyl group, an alkyl group, an alkoxy group, an aryl group, or a halogen atom, where the alkyl groups and alkoxy groups of R¹ to R⁸ may be optionally substituted.

The alkyl groups represented by R¹ to R⁸ may be linear, branched, or cyclic. Preferably, the alkyl groups represented by R¹ to R⁸ are branched.

Examples of the alkyl groups include methyl groups, ethyl groups, n-propyl groups, isopropyl groups, cyclopropyl groups, n-butyl groups, isobutyl groups, sec-butyl groups, tert-butyl groups, cyclobutyl groups, n-pentyl groups, isopentyl groups, neopentyl groups, tert-pentyl groups, cyclopentyl groups, n-hexyl groups, isohexyl groups, cyclohexyl groups, n-heptyl groups, cycloheptanyl groups, n-octyl groups, 2-ethylhexyl groups, cyclooctyl groups, n-nonyl groups, cyclononyl groups, and n-decyl groups.

The alkyl group has preferably 1 to 15 carbon atoms, more preferably 1 to 12 carbon atoms, even more preferably 1 to 10 carbon atoms, particularly preferably 1 to 8 carbon atoms.

The branched or cyclic alkyl group has preferably 3 to 15 carbon atoms, more preferably 3 to 12 carbon atoms, even more preferably 3 to 10 carbon atoms, particularly preferably 3 to 8 carbon atoms.

In view of even superior ambient light stability under high illuminance and even superior photocurability, R¹ is preferably an alkyl group or alkoxy group having 1 to 15 carbon atoms, more preferably a branched alkyl group or alkoxy group having 3 to 15 carbon atoms, even more preferably a 2-ethylhexyloxy group.

The alkoxy groups represented by R¹ to R³ and R⁷ and R⁸ may be linear, branched, or cyclic. Preferably, the alkoxy groups represented by R¹ to R³ and R⁷ and R⁸ are branched.

Examples of the alkoxy groups include methoxy groups, ethoxy groups, n-propoxy groups, isopropoxy groups, n-butoxy groups, tert-butoxy groups, n-hexyloxy groups, cyclohexyloxy groups, n-octyloxy groups, and 2-ethylhexyloxy groups.

The alkoxy group has preferably 1 to 15 carbon atoms, more preferably 1 to 12 carbon atoms, even more preferably 1 to 10 carbon atoms, particularly preferably 1 to 8 carbon atoms.

The branched or cyclic alkoxy group has preferably 3 to 15 carbon atoms, more preferably 3 to 12 carbon atoms, even more preferably 3 to 10 carbon atoms, particularly preferably 3 to 8 carbon atoms.

Examples of the aryl groups represented by R⁷ and R⁸ include phenyl groups, naphthyl groups, and anthryl groups. The aryl group has preferably 6 to 14 carbon atoms, more preferably 6 to 10 carbon atoms.

Examples of the halogen atoms represented by R² and R³ and R⁷ and R⁸ include a fluorine atom, a chlorine atom, a bromine atom, and an iodine atom.

The alkyl groups and alkoxy groups of R¹ to R⁸ may be optionally substituted.

The substituent may be, for example, a halogen atom (a fluorine atom, a chlorine atom, a bromine atom, or an iodine atom), a hydroxyl group, an alkoxy group having 1 to 6 carbon atoms, a dialkylamino group having 1 to 6 carbon atoms on both alkyl groups, or an amino group.

The alkyl groups and alkoxy groups of R¹ to R⁸ may be unsubstituted.

In view of ambient light stability under high illuminance and photocurability, the ultraviolet absorber (A) is preferably a triazine-based ultraviolet absorber represented by the general formula (1) above and having a maximum absorption wavelength in a wavelength range of 310 nm or more and 400 nm or less as measured over a wavelength range of 250 nm to 500 nm.

In view of achieving even greater photocurability while providing even superior stability to ambient light under high illuminance such as operating lights, the ultraviolet absorber (A) exhibits a maximum absorption wavelength in a wavelength range of preferably 390 nm or less, more preferably 385 nm or less, even more preferably 380 nm or less, particularly preferably 375 nm or less.

The ultraviolet absorber (A) is soluble in 2-hydroxyethyl methacrylate at 0.12 parts or more by mass relative to 100 parts by mass of 2-hydroxyethyl methacrylate. It is believed that the use of an ultraviolet absorber having high solubility in monomers allows it to be dispersed throughout the dental composition, ensuring reliable ultraviolet absorption without excess or deficiency, leading to notably superior stability to ambient light under high illuminance. From this perspective, the ultraviolet absorber (A) is soluble in 2-hydroxyethyl methacrylate at preferably 0.14 parts or more by mass, more preferably 0.20 parts or more by mass relative to 100 parts by mass of 2-hydroxyethyl methacrylate.

It should be noted here that the solubility relative to 100 parts by mass of 2-hydroxyethyl methacrylate serves as an indicator for selecting an ultraviolet absorber (A) having high solubility in monomers, and a dental composition of the present invention is not necessarily required to comprise 2-hydroxyethyl methacrylate, as demonstrated in the EXAMPLES below (for example, Examples 1-8 and 2-8).

Specific examples of the ultraviolet absorber (A) include 2,4,6-tris(2,4-dihydroxyphenyl)-1,3,5-triazine, bisethylhexyloxyphenol methoxyphenyl triazine, and 2-[4,6-bis(biphenyl-4-yl)-1,3,5-triazin-2-yl]-5-[(2-ethylhexyl)oxy]phenol. Preferred are bisethylhexyloxyphenol methoxyphenyl triazine and/or 2-[4,6-bis(biphenyl-4-yl)-1,3,5-triazin-2-yl]-5-[(2-ethylhexyl)oxy]phenol.

The ultraviolet absorber (A) may be used alone, or two or more thereof may be used in combination.

In view of even superior stability to ambient light under high illuminance and even superior photocurability due to the structural features of the triazine-based ultraviolet absorber (A) (e.g., steric hindrance attributable to phenyl groups) and the maximum absorption wavelength occurring in a wavelength range of 310 nm or more and 400 nm or less in the triazine-based ultraviolet absorber (A) itself, the content of ultraviolet absorber (A) is preferably 85 parts or more by mass, more preferably 90 parts or more by mass, even more preferably 95 parts or more by mass, yet more preferably 98 parts or more by mass, particularly preferably 100 parts by mass in total 100 parts by mass of the ultraviolet absorbers contained in a dental composition of the present invention.

In view of ambient light stability under high illuminance and photocurability, the content of ultraviolet absorber (A) is preferably 0.0001 mass% or more, more preferably 0.001 mass% or more, even more preferably 0.005 mass% or more, particularly preferably 0.01 mass% or more in 100 mass% of the total mass of a dental composition of the present invention.

In view of ambient light stability under high illuminance and photocurability, the content of ultraviolet absorber (A) is preferably 1.5 mass% or less, more preferably 1.2 mass% or less, even more preferably 0.8 mass% or less, particularly preferably 0.4 mass% or less in 100 mass% of the total mass of a dental composition of the present invention.

In view of ambient light stability under high illuminance and photocurability, the content of ultraviolet absorber (A) is preferably 0.001 parts or more by mass, more preferably 0.01 parts or more by mass, even more preferably 0.05 parts or more by mass, particularly preferably 0.1 parts or more by mass relative to total 100 parts by mass of polymerizable monomer (B).

In view of ambient light stability under high illuminance and photocurability, the content of ultraviolet absorber (A) is preferably 5 parts or less by mass, more preferably 1 part or less by mass, even more preferably 0.8 parts or less by mass, particularly preferably 0.5 parts or less by mass relative to total 100 parts by mass of polymerizable monomer (B).

### [Polymerizable Monomer (B)]

Preferred for use as the polymerizable monomer (B) used in a dental composition of the present invention are radical polymerizable monomers.

Examples of radical polymerizable monomers as polymerizable monomer (B) include (meth)acrylate polymerizable monomers, (meth)acrylamide polymerizable monomers, and esters, vinyl esters, and vinyl ethers of acids such as α-cyanoacrylic acid, (meth)acrylic acid, α-halogenated acrylic acid, crotonic acid, cinnamic acid, sorbic acid, maleic acid, and itaconic acid, as well as mono-N-vinyl derivatives and styrene derivatives. In view of curability, preferred among these are (meth)acrylate polymerizable monomers and (meth)acrylamide polymerizable monomers.

The polymerizable monomer (B) can be broadly classified into polymerizable monomer having an acidic group (B-1), and polymerizable monomer having no acidic group (B-2).

The polymerizable monomer (B) may be used alone, or two or more thereof may be used in combination.

### <Polymerizable Monomer Having Acidic Group (B-1)>

A dental composition of the present invention may comprise a polymerizable monomer having an acidic group (B-1).

The polymerizable monomer having an acidic group (B-1) used in the present invention is preferably a radical polymerizable monomer having at least one acidic group and polymerizable group. In view of adhesive properties, the acidic group is preferably, for example, a phosphoric acid group, a pyrophosphoric acid group, a thiophosphoric acid group, a phosphonic acid group, a carboxylic acid group, or a sulfonic acid group.

In view of photocurability and ease of radical polymerization, preferred as polymerizable groups are (meth)acryloyloxy groups and/or (meth)acrylamide groups.

The polymerizable monomer having an acidic group (B-1) may be incorporated alone, or two or more thereof may be used in combination.

Examples of polymerizable monomers having a phosphoric acid group include 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl] hydrogen phosphate, bis[4-(meth)acryloyloxybutyl] hydrogen phosphate, bis[6-(meth)acryloyloxyhexyl] hydrogen phosphate, bis[8-(meth)acryloyloxyoctyl] hydrogen phosphate, bis[9-(meth)acryloyloxynonyl] hydrogen phosphate, bis[10-(meth)acryloyloxydecyl] hydrogen phosphate, 1,3-di(meth)acryloyloxypropyl dihydrogen phosphate, 2-(meth)acryloyloxyethylphenyl hydrogen phosphate, 2-(meth)acryloyloxyethyl-(2-bromoethyl) hydrogen phosphate, 2-(meth)acryloyloxyethyl-(4-methoxyphenyl) hydrogen phosphate, and 2-(meth)acryloyloxypropyl-(4-methoxyphenyl) hydrogen phosphate, and acid chlorides, alkali metal salts, and amine salts of these.

Examples of polymerizable monomers having a pyrophosphoric acid group include bis[2-(meth)acryloyloxyethyl] pyrophosphate, bis[4-(meth)acryloyloxybutyl] pyrophosphate, bis[6-(meth)acryloyloxyhexyl] pyrophosphate, bis[8-(meth)acryloyloxyoctyl] pyrophosphate, and bis[10-(meth)acryloyloxydecyl] pyrophosphate, and acid chlorides, alkali metal salts, and amine salts of these.

Examples of polymerizable monomers having a thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, and 20-(meth)acryloyloxyicosyl dihydrogen thiophosphate, and acid chlorides, alkali metal salts, and ammonium salts of these.

Examples of polymerizable monomers having a phosphonic acid group include 2-(meth)acryloyloxyethylphenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexylphosphonoacetate, and 10-(meth)acryloyloxydecyl-3-phosphonoacetate, and acid chlorides, alkali metal salts, and ammonium salts of these.

Examples of polymerizable monomers having a carboxylic acid group include monofunctional (meth)acrylic acid esters having one carboxyl group or an acid anhydride group thereof within the molecule, monofunctional (meth)acrylic acid esters having multiple carboxyl groups or acid anhydride groups thereof within the molecule, and acid chlorides, alkali metal salts, and ammonium salts of these.

Examples of the monofunctional polymerizable monomers having one carboxyl group or an acid anhydride group thereof within the molecule include (meth)acrylic acid, N-(meth)acryloyl glycine, N-(meth)acryloyl aspartic acid, 2-(meth)acryloyloxyethyl hydrogen succinate, 2-(meth)acryloyloxyethyl hydrogen phthalate, 2-(meth)acryloyloxyethyl hydrogen malate, O-(meth)acryloyl tyrosine, N-(meth)acryloyl tyrosine, N-(meth)acryloyl phenylalanine, N-(meth)acryloyl-p-aminobenzoic acid, N-(meth)acryloyl-o-aminobenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, and compounds derived by converting the carboxyl group of the aforementioned compounds into an acid anhydride group.

Examples of the monofunctional polymerizable monomers having multiple carboxyl groups or acid anhydride groups thereof within the molecule include 6-(meth)acryloyloxyhexane-1,1-dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1,1-dicarboxylic acid, 12-(meth)acryloyloxydodecane-1,1-dicarboxylic acid, 13-(meth)acryloyloxytridecane-1,1-dicarboxylic acid, 4-(meth)acryloyloxyethyl trimellitate, 4-(meth)acryloyloxyethyl trimellitate anhydride, 4-(meth)acryloyloxybutyl trimellitate, 4-(meth)acryloyloxyhexyl trimellitate, 4-(meth)acryloyloxydecyl trimellitate, 2-(meth)acryloyloxyethyl-3'-(meth)acryloyloxy-2'-(3,4-dicarboxybenzoyloxy)propyl succinate, 6-(meth)acryloyloxyethylnaphthalene-1,2,6-tricarboxylic anhydride, 6-(meth)acryloyloxyethylnaphthalene-2,3,6-tricarboxylic anhydride, 4-(meth)acryloyloxyethylcarbonylpropionoyl-1,8-naphthalic anhydride, and 4-(meth)acryloyloxyethylnaphthalene-1,8-tricarboxylic anhydride.

Examples of polymerizable monomers having a sulfonic acid group include 2-sulfoethyl (meth)acrylate, and acid chlorides, alkali metal salts, and ammonium salts thereof.

In view of providing favorable bond strength as a dental composition, preferred among these polymerizable monomers having an acidic group (B-1) are polymerizable monomers having a phosphoric acid group, or polymerizable monomers having a carboxylic acid group, more preferably 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, 4-(meth)acryloyloxyethyl trimellitate anhydride, 4-(meth)acryloyloxyethyl trimellitate, 11-(meth)acryloyloxyundecane-1,1-dicarboxylic acid, and a mixture of 2-(meth)acryloyloxyethyl dihydrogen phosphate and bis(2-methacryloyloxyethyl) hydrogen phosphate, even more preferably 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, and 20-(meth)acryloyloxyicosyl dihydrogen phosphate. In view of a balance with curability, 10-(meth)acryloyloxydecyl dihydrogen phosphate is most preferred.

The content of the polymerizable monomer having an acidic group (B-1) in a dental composition of the present invention is preferably 0 parts or more by mass and 35 parts or less by mass in total 100 parts by mass of polymerizable monomer (B). In view of adhesive properties to tooth structure, the content of polymerizable monomer having an acidic group (B-1) is more preferably 1 part or more by mass and 30 parts or less by mass, even more preferably 5 parts or more by mass and 25 parts or less by mass, particularly preferably 5 parts or more by mass and 20 parts or less by mass.

### <Polymerizable Monomer Having no Acidic Group (B-2)>

In the present invention, the polymerizable monomer having no acidic group (B-2) can be classified into hydrophilic polymerizable monomer having no acidic group and that has a solubility of 4.5 g/L or more in 25°C water (B-2a), and hydrophobic polymerizable monomer having no acidic group and that has a solubility of less than 4.5 g/L in 25°C water (B-2b).

The polymerizable monomer having no acidic group (B-2) may be incorporated alone, or two or more thereof may be used in combination.

A certain preferred embodiment is, for example, a dental composition in which the polymerizable monomer (B) comprises a polymerizable monomer having no acidic group (B-2), and the polymerizable monomer having no acidic group (B-2) comprises a hydrophilic polymerizable monomer having no acidic group and that has a solubility of 4.5 g/L or more in 25°C water (B-2a), and/or a hydrophobic polymerizable monomer having no acidic group and that has a solubility of less than 4.5 g/L in 25°C water (B-2b).

### · Hydrophilic Polymerizable Monomer Having no Acidic Group (B-2a)

In a dental composition of the present invention, the hydrophilic polymerizable monomer having no acidic group (B-2a) (hereinafter, also referred to simply as "hydrophilic polymerizable monomer (B-2a)") in the polymerizable monomer (B) refers to monomers that do not have acidic groups and have a solubility of 4.5 g/L or more in 25°C water.

The hydrophilic polymerizable monomer (B-2a) may be used alone, or two or more thereof may be used in combination.

The hydrophilic polymerizable monomer (B-2a) improves the wettability of the dental composition to tooth structure. Preferred as hydrophilic polymerizable monomer (B-2a) are radical polymerizable monomers lacking acidic groups and having polymerizable groups. In view of ease of radical polymerization, the polymerizable groups are preferably (meth)acryloyloxy groups and/or (meth)acrylamide groups.

Examples of the hydrophilic polymerizable monomer (B-2a) include:
hydrophilic (meth)acrylate monofunctional polymerizable monomers such as 2-hydroxyethyl (meth)acrylate, 3-hydroxypropyl (meth)acrylate, 2-hydroxypropyl (meth)acrylate, 1,3-dihydroxypropyl (meth)acrylate, 2,3-dihydroxypropyl (meth)acrylate, 2-((meth)acryloyloxy)ethyltrimethylammonium chloride, and tetrahydrofurfuryl (meth)acrylate;
hydrophilic (meth)acrylate polyfunctional polymerizable monomers such as triethylene glycol di(meth)acrylate, tetraethylene glycol di(meth)acrylate, nonaethylene glycol di(meth)acrylate, 1,3-bis(methacryloyloxy)-2-propanol, and 1,2-bis(3-methacryloyloxy-2-hydroxypropoxy)ethane; and
hydrophilic (meth)acrylamide polymerizable monomers such as N-methylol(meth)acrylamide, N-hydroxyethyl(meth)acrylamide, N,N-bis(2-hydroxyethyl)(meth)acrylamide, N-methoxymethyl(meth)acrylamide, N-ethoxymethyl(meth)acrylamide, diacetone(meth)acrylamide, 4-(meth)acryloylmorpholine, N-trihydroxymethyl-N-methyl(meth)acrylamide, N,N-dimethylacrylamide, N,N-diethylacrylamide, and N-methacryloyloxyethylacrylamide.

In view of hydrophilicity and curability, preferred for use among these hydrophilic polymerizable monomers (B-2a) are (meth)acrylate polyfunctional polymerizable monomers, more preferably 2-hydroxyethyl methacrylate (commonly known as HEMA), N,N-diethylacrylamide (commonly known as DEAA), triethylene glycol dimethacrylate (commonly known as 3G), 1,3-bis(methacryloyloxy)-2-propanol, and 1,2-bis(3-methacryloyloxy-2-hydroxypropoxy)ethane (commonly known as #801), even more preferably 3G and #801.

The content of the hydrophilic polymerizable monomer (B-2a) in a dental composition of the present invention is preferably 0 parts or more by mass in total 100 parts by mass of polymerizable monomer (B). In view of adhesive properties, the content of hydrophilic polymerizable monomer (B-2a) is more preferably 5 parts or more by mass, even more preferably 10 parts or more by mass, particularly preferably 15 parts or more by mass.

In view of adhesive properties, the content of hydrophilic polymerizable monomer (B-2a) is preferably 60 parts or less by mass, more preferably 50 parts or less by mass, even more preferably 45 parts or less by mass, particularly preferably 40 parts or less by mass in total 100 parts by mass of polymerizable monomer (B).

When using a dental composition of the present invention as a dental bonding material, it is preferable to comprise a hydrophilic polymerizable monomer (B-2a). In view of adhesive properties, when using a dental composition of the present invention as a dental bonding material, the content of hydrophilic polymerizable monomer (B-2a) is preferably 5 parts or more by mass and 60 parts or less by mass in total 100 parts by mass of polymerizable monomer (B).

When using a dental composition of the present invention as a dental composite resin, the hydrophilic polymerizable monomer (B-2a) may be absent. That is, the content of hydrophilic polymerizable monomer (B-2a) may be 0 parts by mass in total 100 parts by mass of polymerizable monomer (B).

### · Hydrophobic Polymerizable Monomer Having no Acidic Group (B-2b)

In a dental composition of the present invention, in view of properties such as the mechanical strength of the cured product, it is preferable that the polymerizable monomer (B) comprise a hydrophobic polymerizable monomer having no acidic group (B-2b) (hereinafter, also referred to simply as "hydrophobic polymerizable monomer (B-2b)").

The hydrophobic polymerizable monomer (B-2b) refers to monomers that do not have acidic groups and have a solubility of less than 4.5 g/L in 25°C water.

By using the hydrophobic polymerizable monomer (B-2b), it is possible to improve properties such as mechanical strength in the cured product of the dental composition. Additionally, stringiness (stringing tendency) can be reduced in dental composite resin applications.

The hydrophobic polymerizable monomer (B-2b) may be incorporated alone, or two or more thereof may be used in combination.

Preferred as hydrophobic polymerizable monomer (B-2b) are radical polymerizable monomers lacking acidic groups and having polymerizable groups. In view of ease of radical polymerization, the polymerizable groups are preferably (meth)acryloyloxy groups and/or (meth)acrylamide groups.

Examples of the hydrophobic polymerizable monomer (B-2b) include hydrophobic monofunctional polymerizable monomers, and crosslinkable polymerizable monomers such as hydrophobic aromatic bifunctional polymerizable monomers, hydrophobic aliphatic bifunctional polymerizable monomers, and hydrophobic tri- and higher-functional polymerizable monomers. Other examples include polyfunctional polymerizable monomers containing a urethane bond and a polymer backbone.

In this specification, the hydrophobic aromatic bifunctional polymerizable monomers, hydrophobic aliphatic bifunctional polymerizable monomers, and hydrophobic tri- and higher-functional polymerizable monomers are regarded as polyfunctional polymerizable monomers containing a urethane bond and a polymer backbone when they fall under the category of polyfunctional polymerizable monomers containing a urethane bond and a polymer backbone. Stated differently, the hydrophobic aromatic bifunctional polymerizable monomers, hydrophobic aliphatic bifunctional polymerizable monomers, and hydrophobic tri- and higher-functional polymerizable monomers exclude polyfunctional polymerizable monomers containing a urethane bond and a polymer backbone.

Examples of the hydrophobic monofunctional polymerizable monomers include:
aliphatic monofunctional (meth)acrylate monomers such as methyl (meth)acrylate, ethyl (meth)acrylate, butyl (meth)acrylate, and n-stearyl methacrylate;
aliphatic monofunctional (meth)acrylate monomers containing an ether bond, such as butoxydiethylene glycol methacrylate, and methoxypolyethylene glycol methacrylate (with an average of 9 moles of oxyethylene group added);
alicyclic monofunctional (meth)acrylate monomers such as cyclohexyl methacrylate, isobornyl methacrylate, and dicyclopentanyl methacrylate;
monofunctional (meth)acrylate monomers having an aromatic ring group, such as p-cumyl-phenoxyethylene glycol (meth)acrylate, benzyl (meth)acrylate, phenoxybenzyl (meth)acrylate, and 2-phenoxyethyl (meth)acrylate; and
(meth)acrylate monomers containing a heterocyclic ring group (for example, a cyclic ether group), such as tetrahydrofurfuryl (meth)acrylate.

The monofunctional (meth)acrylate monomers having an aromatic ring group are preferably those having one or two phenyl groups.

The (meth)acrylate monomers containing a heterocyclic ring group are preferably those having one or two heterocyclic ring groups (for example, cyclic ether groups).

In view of the mechanical strength of the cured product, preferred among these are tetrahydrofurfuryl methacrylate (commonly known as THF-MA), benzyl methacrylate (commonly known as BEMA), phenoxybenzyl methacrylate (commonly known as POB-MA), and 2-phenoxyethyl methacrylate (commonly known as PEMA).

Examples of the hydrophobic aromatic bifunctional polymerizable monomers include 2,2-bis((meth)acryloyloxyphenyl)propane, 2,2-bis[4-(2-hydroxy-3-(meth)acryloyloxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane. Preferred among these are 2,2-bis[4-(2-hydroxy-3-methacryloyloxypropoxy)phenyl]propane (commonly known as Bis-GMA), 2,2-bis(4-methacryloyloxyethoxyphenyl)propane, 2,2-bis(4-methacryloyloxypolyethoxyphenyl)propane (with an average of 2.6 moles of ethoxy group added; commonly known as D-2.6E), 2,2-bis(4-methacryloyloxydiethoxyphenyl)propane, 2,2-bis(4-methacryloyloxytriethoxyphenyl)propane, 2,2-bis(4-methacryloyloxytetraethoxyphenyl)propane, and 2,2-bis(4-methacryloyloxypentaethoxyphenyl)propane. More preferred are Bis-GMA and D-2.6E.

Examples of the aliphatic bifunctional polymerizable monomers include monoethylene glycol di(meth)acrylate, diethylene glycol di(meth)acrylate, propylene glycol di(meth)acrylate, butylene glycol di(meth)acrylate, neopentyl glycol di(meth)acrylate, 1,5-pentanediol di(meth)acrylate, 1,6-hexanediol di(meth)acrylate, 1,10-decanediol di(meth)acrylate, and 2,2,4-trimethylhexamethylenebis(2-carbamoyloxyethyl) di(meth)acrylate. Preferred among these are neopentyl glycol dimethacrylate (commonly known as NPG), 2,2,4-trimethylhexamethylenebis(2-carbamoyloxyethyl) dimethacrylate (commonly known as UDMA), N-methacryloyloxyethylacrylamide (commonly known as MAEA), 1,10-decanediol dimethacrylate (commonly known as DD), and 2,2,4-trimethylhexamethylenebis(2-carbamoyloxyethyl) dimethacrylate. More preferred are NPG, UDMA, and DD.

Examples of the tri- and higher-functional polymerizable monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acrylate, and 1,7-diacryloyloxy-2,2,6,6-tetra(meth)acryloyloxymethyl-4-oxaheptane. Preferred among these is N,N-(2,2,4-trimethylhexamethylene)bis[2-(aminocarboxy)propane-1,3-diol]tetramethacrylate.

Examples of the polyfunctional polymerizable monomers having a urethane bond and a polymer backbone include urethanized (meth)acrylate polymerizable monomers having incorporated a urethane bond adjacent a (meth)acryloyloxy group and possessing, within a single molecule, at least one structure (also referred to as "polymer backbone" hereinbelow) selected from the group consisting of a polyester, a polycarbonate, a polyurethane, a polyether, a poly-conjugated diene, and a hydrogenated poly-conjugated diene. In view of superior flexibility, preferred are those containing an aromatic ring in the repeating units forming the polymer backbone.

As an example, a urethanized (meth)acrylate polyfunctional polymerizable monomer having a polymer backbone can be easily synthesized through an addition reaction between a polyol having a polymer backbone, a compound having an isocyanate group (-NCO), and a (meth)acrylate compound having a hydroxyl group (-OH). A urethanized (meth)acrylate polyfunctional polymerizable monomer having a polymer backbone can also be easily synthesized by allowing lactone or alkylene oxide to undergo a ring-opening addition reaction with a (meth)acrylate compound having a hydroxyl group, and causing the resulting compound with a terminal hydroxyl group to undergo an addition reaction with a compound having an isocyanate group.

The polyol having a polymer backbone is not particularly limited, as long as the above structure is present. In the case of a polyester, examples include a polymer of phthalic acid and an alkylene diol having 2 to 12 carbon atoms, a polymer of adipic acid and an alkylene glycol having 2 to 12 carbon atoms, a polymer of maleic acid and an alkylene diol having 2 to 12 carbon atoms, a β-propiolactone polymer, a γ-butyrolactone polymer, a δ-valerolactone polymer, an ε-caprolactone polymer, and copolymers of these.

Examples of the polycarbonate include a polycarbonate derived from a C2 to C12 aliphatic diol, a polycarbonate derived from bisphenol A, and a polycarbonate derived from a C2 to C12 aliphatic diol and bisphenol A.

Examples of the polyurethane include a polymer of a C2 to C12 aliphatic diol and a C1 to C12 diisocyanate.

Examples of the polyether include polyethylene glycol, polypropylene glycol, polybutylene glycol, and poly(1-methylbutylene glycol).

Examples of the poly-conjugated diene and the hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these.

Examples of the compound having an isocyanate group include hexamethylene diisocyanate (HDI), tolylene diisocyanate (TDI), xylylene diisocyanate (XDI), diphenylmethane diisocyanate (MDI), isophorone diisocyanate (IPDI), trimethylhexamethylene diisocyanate (TMHMDI), tricyclodecane diisocyanate (TCDDI), and adamantane diisocyanate (ADI).

Examples of the (meth)acrylate compound having a hydroxyl group include hydroxy (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis[4-[3-(meth)acryloyloxy-2-hydroxypropoxy]phenyl]propane, 1,2-bis[3-(meth)acryloyloxy-2-hydroxypropoxy]ethane, pentaerythritol tri(meth)acrylate, and tri or tetra(meth)acrylates of dipentaerythritol.

The addition reaction between a compound having an isocyanate group and a (meth)acrylate compound having a hydroxyl group can be carried out following a known method, and is not particularly limited.

The urethanized (meth)acrylate polyfunctional polymerizable monomer having a polymer backbone that can be obtained using the above method may be the product of a reaction involving any combination of the foregoing compounds: a polyol having at least one structure selected from the group consisting of a polyester, a polycarbonate, a polyurethane, a polyether, a poly-conjugated diene, and a hydrogenated poly-conjugated diene; a compound having an isocyanate group; and a (meth)acrylate compound having a hydroxyl group. The urethanized (meth)acrylate polyfunctional polymerizable monomer having a polymer backbone may be a commercially available product. Examples of commercially available products include urethane acrylates such as UFC-01 (manufactured by Kyoeisha Chemical Co., Ltd.), EBECRYL 8465 (manufactured by DAICEL-ALLNEX LTD.), and UN 7600 (manufactured by Negami Chemical Industrial Co., Ltd.).

In view of viscosity and strength, the urethanized (meth)acrylate polyfunctional polymerizable monomer having a polymer backbone has a weight-average molecular weight (Mw) of preferably 500 to 50,000, more preferably 750 to 30,000, even more preferably 1,000 to 15,000.

In the present invention, the weight-average molecular weight (Mw) means a weight-average molecular weight in terms of polystyrene equivalents, as determined by gel permeation chromatography (GPC).

In view of handling properties and the mechanical strength of the cured product, preferred among these hydrophobic polymerizable monomers (B-2b) are aromatic bifunctional polymerizable monomers, and aliphatic bifunctional polymerizable monomers.

When the content of the hydrophobic polymerizable monomer (B-2b) in a dental composition of the present invention is excessively high, it may lead to a decrease in the wettability of the dental composition to tooth structure, resulting in reduced adhesion. In contrast, an excessively low content of hydrophobic polymerizable monomer (B-2b) may result in insufficient mechanical strength in the cured product.

Accordingly, the content of the hydrophobic polymerizable monomer (B-2b) in a dental composition of the present invention is preferably 40 parts or more by mass, more preferably 45 parts or more by mass, even more preferably 50 parts or more by mass, particularly preferably 55 parts or more by mass in total 100 parts by mass of polymerizable monomer (B).

### [Photopolymerization Initiator (C)]

A dental composition of the present invention comprises a photopolymerization initiator (C).

The photopolymerization initiator (C) is not particularly limited, and any known photopolymerization initiators can be used without restrictions. Examples of photopolymerization initiator (C) include α-diketones, ketals, thioxanthones, (bis)acylphosphine oxides, and α-aminoacetophenones.

The photopolymerization initiator (C) may be used alone, or two or more thereof may be used in combination.

Examples of α-diketones include dl-camphorquinone (commonly known as CQ), benzil, and 2,3-pentanedione.

Examples of ketals include benzyl dimethyl ketal, and benzyl diethyl ketal.

Examples of thioxanthones include 2-chlorothioxanthone, and 2,4-diethylthioxanthone.

Examples of (bis)acylphosphine oxides include 2,4,6-trimethylbenzoyldiphenylphosphine oxide, bis(2,4,6-trimethylbenzoyl)phenylphosphine oxide, dibenzoylphenylphosphine oxide, bis(2,6-dimethoxybenzoyl)phenylphosphine oxide, tris(2,4-dimethylbenzoyl)phosphine oxide, tris(2-methoxybenzoyl)phosphine oxide, 2,6-dimethoxybenzoyldiphenylphosphine oxide, 2,6-dichlorobenzoyldiphenylphosphine oxide, 2,3,5,6-tetramethylbenzoyldiphenylphosphine oxide, benzoyl-bis(2,6-dimethylphenyl)phosphine oxide, 2,4,6-trimethylbenzoylethoxyphenylphosphine oxide, the water-soluble acylphosphine oxide compounds disclosed in JP H03-57916 B, and salts of these (for example, alkali metal salts such as sodium salts (e.g., sodium bis(2,4,6-trimethylbenzoyl)phosphinate)).

Examples of α-aminoacetophenones include 2-benzyl-2-dimethylamino-1-(4-morpholinophenyl)-1-butanone, 2-benzyl-2-diethylamino-1-(4-morpholinophenyl)-1-butanone, 2-benzyl-2-dimethylamino-1-(4-morpholinophenyl)-1-propanone, 2-benzyl-2-diethylamino-1-(4-morpholinophenyl)-1-propanone, 2-benzyl-2-dimethylamino-1-(4-morpholinophenyl)-1-pentanone, and 2-benzyl-2-diethylamino-1-(4-morpholinophenyl)-1-pentanone.

In view of properties such as curability, preferred as photopolymerization initiator (C) are α-diketones and/or (bis)acylphosphine oxides, more preferably α-diketones, even more preferably dl-camphorquinone.

In view of curability and storage stability, the content of photopolymerization initiator (C) is preferably 0.005 parts or more by mass, more preferably 0.01 parts or more by mass, even more preferably 0.1 parts or more by mass relative to total 100 parts by mass of polymerizable monomer (B).

In view of curability and storage stability, the content of photopolymerization initiator (C) is preferably 10 parts or less by mass, more preferably 5 parts or less by mass, even more preferably 3 parts or less by mass.

The mass ratio (A):(C) of the content of ultraviolet absorber (A) and the content of photopolymerization initiator (C) is preferably 1:0.01 to 1:80. In view of providing even superior ambient light stability under high illuminance and even superior photocurability while retaining excellent storage stability, the mass ratio (A):(C) is more preferably 1:0.1 to 1:50, even more preferably 1:0.5 to 1:30, particularly preferably 1:1 to 1:25, most preferably 1:1.5 to 1:25.

### [Polymerization Accelerator (D)]

Preferably, a dental composition of the present invention further comprises a polymerization accelerator (D).

The polymerization accelerator (D) is not particularly limited. Examples include amines, aldehydes, and thiol compounds.

The polymerization accelerator (D) may be used alone, or two or more thereof may be used in combination.

Examples of amines include aliphatic amines, and aromatic amines.

Examples of aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl (meth)acrylate, N-methyldiethanolamine di(meth)acrylate, N-ethyldiethanolamine di(meth)acrylate, triethanolamine tri(meth)acrylate, triethanolamine, trimethylamine, triethylamine, and tributylamine.

In view of photocurability, preferred among these are tertiary aliphatic amines, particularly preferably N-methyldiethanolamine, triethanolamine, and 2-(dimethylamino)ethyl methacrylate.

Examples of aromatic amines include aromatic amines having an electron withdrawing group on the aromatic ring, and aromatic amines having no electron withdrawing group on the aromatic ring.

Examples of aromatic amines having an electron withdrawing group on the aromatic ring include tertiary aromatic amines having an electron withdrawing group on the aromatic ring, such as n-butoxyethyl 4-(N,N-dimethylamino)benzoate, (2-methacryloyloxy)ethyl 4-(N,N-dimethylamino)benzoate, ethyl 4-(N,N-dimethylamino)benzoate (hereinafter, also referred to by the abbreviation "DABE"), butyl 4-(N,N-dimethylamino)benzoate, and 4-(N,N-dimethylamino)benzophenone.

Examples of aromatic amines having no electron withdrawing group on the aromatic ring include aromatic tertiary amines having no electron withdrawing group on the aromatic ring, such as N,N-bis(2-hydroxyethyl)-3,5-dimethylaniline, N,N-bis(2-hydroxyethyl)-p-toluidine (hereinafter, also referred to by the abbreviation "DEPT"), N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, and N,N-dimethyl-3,5-di-t-butylaniline.

In view of the ability to impart superior curability to the dental composition, preferred is at least one selected from the group consisting of N,N-bis(2-hydroxyethyl)-p-toluidine, ethyl 4-(N,N-dimethylamino)benzoate, n-butoxyethyl 4-(N,N-dimethylamino)benzoate, and 4-(N,N-dimethylamino)benzophenone.

Examples of aldehydes include terephthalaldehyde, and benzaldehyde derivatives. Examples of benzaldehyde derivatives include dimethylaminobenzaldehyde, p-methoxybenzaldehyde, p-ethoxybenzaldehyde, and p-n-octyloxybenzaldehyde.

Examples of thiol compounds include 3-mercaptopropyltrimethoxysilane, 2-mercaptobenzoxazole, decanethiol, and thiobenzoic acid.

In view of curability and storage stability, the content of polymerization accelerator (D) is preferably 0.001 to 20 parts by mass, more preferably 0.01 to 15 parts by mass, even more preferably 0.1 to 10 parts by mass relative to total 100 parts by mass of polymerizable monomer (B).

### [Filler (E)]

Preferably, a dental composition of the present invention further comprises a filler (E).

The filler (E) may be any filler, provided that it does not hinder the effectiveness of the present invention. Examples include inorganic fillers, organic fillers, and composite fillers of inorganic fillers and organic fillers.

The filler (E) may be used alone, or two or more thereof may be used in combination.

Examples of inorganic fillers include the following:
various types of glass (containing silica as a main component, and, optionally, oxides of elements such as heavy metals, boron, and aluminum), including, for example, glass powders of common compositions, such as fused silica, quartz, soda-lime-silica glass, E glass, C glass, and borosilicate glass (PYREX^{®} glass), and dental glass powders such as barium glass (e.g., GM27884 and 8235 manufactured by SCHOTT, and E-2000 and E-3000 manufactured by ESSTECH), strontium·borosilicate glass (e.g., E-4000 manufactured by ESSTECH), lanthanum glass-ceramic (e.g., GM31684 manufactured by SCHOTT), and fluoroaluminosilicate glass (e.g., GM35429, G018-091, and G018-117 manufactured by SCHOTT); and
various types of ceramics, composite oxides (such as silica-titania, and silica-zirconia), diatomaceous earth, kaolin, clay minerals (such as montmorillonite), activated earth, synthetic zeolite, mica, calcium fluoride, ytterbium fluoride, yttrium fluoride, core-shell structured calcium fluoride with silica coating, core-shell structured ytterbium fluoride with silica coating, core-shell structured yttrium fluoride with silica coating, calcium phosphate, barium sulfate, zirconium dioxide, titanium dioxide, hydroxyapatite, core-shell structured calcium phosphate with silica coating, core-shell structured barium sulfate with silica coating, core-shell structured zirconium dioxide with silica coating, core-shell structured titanium dioxide with silica coating, and core-shell structured hydroxyapatite with silica coating.

In view of strength and other properties, preferred among these are various types of glass, composite oxides (such as silica-titania, and silica-zirconia), core-shell structured calcium fluoride with silica coating, core-shell structured ytterbium fluoride with silica coating, core-shell structured yttrium fluoride with silica coating, core-shell structured calcium phosphate with silica coating, core-shell structured barium sulfate with silica coating, core-shell structured zirconium dioxide with silica coating, core-shell structured titanium dioxide with silica coating, and core-shell structured hydroxyapatite with silica coating.

In view of adhesive properties and handling properties, the inorganic filler is preferably a particulate silica with an average primary particle diameter of 0.001 to 10 µm.

Examples of commercially available products include Aerosil^{®} OX50, Aerosil^{®} 50, Aerosil^{®} 200, Aerosil^{®} 380, Aerosil^{®} R972, and Aerosil^{®} 130 (all manufactured by Nippon Aerosil Co., Ltd. under these trade names).

In the present invention, the average particle diameter of inorganic fillers means the average particle diameter before surface treatment when the inorganic filler is surface treated as in the descriptions below.

Examples of organic fillers include polymethyl methacrylate, polyethyl methacrylate, a methyl methacrylate-ethyl methacrylate copolymer, crosslinked polymethyl methacrylate, crosslinked polyethyl methacrylate, polyamide, polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer.

Examples of inorganic-organic composite fillers include those with an inorganic filler dispersed in an organic filler, and inorganic-organic composite fillers where the inorganic filler is coated with various polymers.

In this specification, the average particle diameter of filler (E) can be determined by a laser diffraction scattering method or electron microscopy of particles. Specifically, a laser diffraction scattering method is more convenient for the measurement of particles with a particle size of 0.1 µm or more, whereas electron microscopy is a more convenient method of particle size measurement for ultrafine particles of less than 0.1 µm. Here, 0.1 µm is a measured value by a laser diffraction scattering method.

As a specific example of a laser diffraction scattering method, the particle size may be measured by volume using, for example, a laser diffraction particle size distribution analyzer (Model SALD-2300, manufactured by Shimadzu Corporation) with a 0.2% sodium hexametaphosphate aqueous solution used as dispersion medium.

In electron microscopy, for example, filler particles may be photographed with a scanning electron microscope (Model S-4000, manufactured by Hitachi, Ltd.), and the size of particles (at least 200 particles) observed in a unit field of the captured image may be measured using image-analyzing particle size distribution measurement software (Mac-View, manufactured by Mountech Co., Ltd.). Here, the particle diameter is determined as an arithmetic mean value of the maximum and minimum lengths of particles, and the average particle diameter is calculated from the number of particles and the particle diameter.

In view of curability, when using a dental composition of the present invention as a dental bonding material, the content of filler (E) is preferably 0 mass% or more and 30 mass% or less, more preferably 0.5 mass% or more and 20 mass% or less, particularly preferably 1 mass% or more and 10 mass% or less in 100 mass% of the total mass of the dental composition.

In view of curability, when using a dental composition of the present invention as a dental composite resin, the content of filler (E) is preferably 50 to 90 mass%, more preferably 60 to 80 mass% in 100 mass% of the total mass of the dental composition.

In order to improve curability, mechanical strength, and handling properties, the filler (E) may be used after surface treatment with a known surface treatment agent such as a silane coupling agent.

Examples of the surface treatment agent include silane coupling agents such as vinyltrimethoxysilane, vinyltriethoxysilane, vinyltrichlorosilane, vinyltri(β-methoxyethoxy)silane, γ-methacryloyloxypropyltrimethoxysilane, γ-glycidoxypropyltrimethoxysilane, γ-mercaptopropyltrimethoxysilane, and γ-aminopropyltriethoxysilane.

A dental composition of the present invention may additionally comprise a silane coupling agent other than a silane coupling agent serving as a surface treatment agent for the filler (E).

The silane coupling agent may be used alone, or two or more thereof may be used in combination.

The silane coupling agent may be a known silane coupling agent, without any restrictions.

Specific examples of the silane coupling agent include vinyltrimethoxysilane, vinyltriethoxysilane, vinyltrichlorosilane, vinyltri(β-methoxyethoxy)silane, γ-methacryloyloxypropyltrimethoxysilane, γ-glycidoxypropyltrimethoxysilane, γ-mercaptopropyltrimethoxysilane, γ-aminopropyltriethoxysilane, 5-(meth)acryloyloxypentyltrimethoxysilane, 6-(meth)acryloyloxyhexyltrimethoxysilane, 7-(meth)acryloyloxyheptyltrimethoxysilane, 8-(meth)acryloyloxyoctyltrimethoxysilane, 9-(meth)acryloyloxynonyltrimethoxysilane, 10-(meth)acryloyloxydecyltrimethoxysilane, 11-(meth)acryloyloxyundecyltrimethoxysilane, 8-(meth)acryloyloxyoctylmethyldimethoxysilane, 10-(meth)acryloyloxydecylmethyldimethoxysilane, 11-(meth)acryloyloxyundecylmethyldimethoxysilane, and (meth)acryloyloxymethylphenethyltrimethoxysilane.

In view of adhesive properties, the content of the silane coupling agent is preferably 0.1 to 10.0 mass%, more preferably 0.5 to 9.0 mass%, even more preferably 1.0 to 8.0 mass%, particularly preferably 1.2 to 7.0 mass% in 100 mass% of the total mass of a dental composition of the present invention.

### [Chemical Polymerization Initiator (F)]

A dental composition of the present invention may comprise a chemical polymerization initiator (F).

Examples of chemical polymerization initiator (F) include peroxides (organic peroxides, inorganic peroxides). These are not particularly limited, and known peroxides may be used.

The chemical polymerization initiator (F) may be used alone, or two or more thereof may be used in combination.

Typical examples of organic peroxides include hydroperoxides, peroxyesters, ketone peroxides, peroxyketals, dialkyl peroxides, diacyl peroxides, and peroxydicarbonates. Particularly preferred among these are hydroperoxides and peroxyesters. In view of reducing variation in working time even after prolonged storage of a dental composition of the present invention, peroxyesters are most preferred.

Specific examples of hydroperoxides include cumene hydroperoxide, t-butyl hydroperoxide, t-hexyl hydroperoxide, p-menthane hydroperoxide, diisopropylbenzene hydroperoxide, and 1,1,3,3-tetramethylbutyl hydroperoxide (hereinafter, also referred to by the abbreviation "THP").

The peroxyesters may be any known peroxyesters with no limitation, provided that the peroxy group (-OO- group) has an acyl group at one end, and a hydrocarbon group (or a similar group) at the other end. Specific examples include α,α-bis(neodecanoylperoxy)diisopropylbenzene, cumyl peroxyneodecanoate, 1,1,3,3-tetramethylbutyl peroxyneodecanoate, 1-cyclohexyl-1-methylethyl peroxyneodecanoate, t-hexyl peroxyneodecanoate, t-butyl peroxyneodecanoate, t-hexyl peroxypivalate, t-butyl peroxypivalate, 1,1,3,3-tetramethylbutyl peroxy-2-ethylhexanoate, 2,5-dimethyl-2,5-bis(2-ethylhexanoylperoxy)hexane, 1-cyclohexyl-1-methylethyl peroxy-2-ethylhexanoate, t-hexyl peroxy-2-ethylhexanoate, t-butyl peroxy-2-ethylhexanoate, t-butyl peroxyisobutyrate, t-hexyl peroxyisopropyl monocarbonate, t-butyl peroxymaleic acid, t-butyl peroxy-3,5,5-trimethylhexanoate, t-butyl peroxylaurate, 2,5-dimethyl-2,5-bis(m-toluoylperoxy)hexane, t-butyl peroxyisopropyl monocarbonate, t-butyl peroxy-2-ethylhexyl monocarbonate, t-hexyl peroxybenzoate, 2,5-dimethyl-2,5-bis(benzoylperoxy)hexane, t-butyl peroxyacetate, t-butyl peroxy-m-toluoylbenzoate, t-butyl peroxybenzoate (hereinafter, also referred to by the abbreviation "BPB"), and bis(t-butylperoxy)isophthalate. In view of storage stability and reactivity, preferred among these are t-butyl peroxymaleic acid, t-butyl peroxy-3,5,5-trimethylhexanoate, t-butyl peroxybenzoate, t-butyl peroxyisopropyl monocarbonate, t-butyl peroxy-2-ethylhexyl monocarbonate, and t-butyl peroxyacetate, more preferably t-butyl peroxybenzoate.

Examples of ketone peroxides include methyl ethyl ketone peroxide, cyclohexanone peroxide, methyl cyclohexanone peroxide, methyl acetoacetate peroxide, and acetyl acetone peroxide.

Examples of peroxyketals include 1,1-bis(t-hexylperoxy)3,3,5-trimethylcyclohexane, 1,1-bis(t-hexylperoxy)cyclohexane, 1,1-bis(t-butylperoxy)3,3,5-trimethylcyclohexanone, 1,1-bis(t-butylperoxy)cyclohexane, 1,1-bis(t-butylperoxy)cyclodecane, 2,2-bis(t-butylperoxy)butane, n-butyl 4,4-bis(t-butylperoxy)valerate, and 2,2-bis(4,4-di-t-butylperoxycyclohexyl)propane.

Examples of dialkyl peroxides include α,α-bis(t-butylperoxy)diisopropylbenzene, dicumyl peroxide, 2,5-dimethyl-2,5-bis(t-butylperoxy)hexane, t-butyl cumyl peroxide, di-t-butyl peroxide, and 2,5-dimethyl-2,5-bis(t-butylperoxy)3-hexyne.

Examples of diacyl peroxides include isobutyryl peroxide, 2,4-dichlorobenzoyl peroxide, 3,5,5-trimethylhexanoyl peroxide, octanoyl peroxide, lauroyl peroxide, stearyl peroxide, succinic acid peroxide, m-toluoylbenzoyl peroxide, and benzoyl peroxide.

Examples of peroxydicarbonates include di-n-propylperoxy dicarbonate, diisopropylperoxy dicarbonate, bis(4-t-butylcyclohexyl)peroxydicarbonate, di(2-ethoxyethyl)peroxydicarbonate, di(2-ethylhexyl)peroxydicarbonate, di(2-methoxybutyl)peroxydicarbonate, and di(3-methyl-3-methoxybutyl)peroxydicarbonate.

Examples of inorganic peroxides include peroxydisulfates and peroxydiphosphates. In view of curability, peroxydisulfates are preferred. Specific examples of peroxydisulfates include sodium peroxydisulfate, potassium peroxydisulfate (hereinafter, also referred to by the abbreviation "KPS"), aluminum peroxydisulfate, and ammonium peroxydisulfate.

In view of curability, the content of organic peroxides and inorganic peroxides is preferably 0.01 to 5 parts by mass, more preferably 0.05 to 2 parts by mass in total 100 parts by mass of the polymerizable monomer (B) in a dental composition of the present invention.

### [Chemical Polymerization Accelerator (G)]

Examples of the chemical polymerization accelerator (G) used in a dental composition of the present invention include period 4 transition metal compounds, transition metal compounds outside of period 4, thiourea compounds, tin compounds, aromatic sulfinic acids and salts thereof, benzotriazole compounds, benzoimidazole compounds, bromides, borate compounds, barbituric acid compounds, ascorbic acid compounds, and sulfur-containing reducing inorganic compounds.

The chemical polymerization accelerator (G) may be used alone, or two or more thereof may be used in combination.

In a certain embodiment, when the polymerizable monomer (B) does not comprise a polymerizable monomer having an acidic group (B-1), the chemical polymerization accelerator (G) is preferably at least one selected from the group consisting of a period 4 transition metal compound, a transition metal compound outside of period 4, and a thiourea compound.

In view of curability and storage stability, the content of chemical polymerization accelerator (G) in such an embodiment is preferably 0.001 to 20 parts by mass, more preferably 0.005 to 10 parts by mass, even more preferably 0.025 to 5 parts by mass relative to total 100 parts by mass of polymerizable monomer (B).

In another certain embodiment, when the polymerizable monomer (B) comprises a polymerizable monomer having an acidic group (B-1), the chemical polymerization accelerator (G) is preferably at least one selected from the group consisting of a copper compound, an aromatic sulfinic acid and a salt thereof, a benzotriazole compound, a benzoimidazole compound, a bromide, an ascorbic acid compound, and a sulfur-containing reducing inorganic compound.

In view of curability and storage stability, the content of chemical polymerization accelerator (G) in such an embodiment is preferably 0.001 to 40 parts by mass, more preferably 0.005 to 20 parts by mass, even more preferably 0.025 to 10 parts by mass relative to total 100 parts by mass of polymerizable monomer (B).

The period 4 transition metal compound may be any of a vanadium compound, a copper compound, or a period 4 transition metal compound excluding vanadium and copper (hereinafter, also referred to simply as "other period 4 transition metal compounds").

In certain embodiments, in view of accelerating polymerization, preferred as chemical polymerization accelerator (G) are vanadium compounds and copper compounds.

Examples of vanadium compounds include vanadyl(IV) acetylacetonate, vanadyl stearate, vanadium naphthenate, vanadium benzoylacetonate, vanadyl oxalate, bis(maltolato)oxovanadium(IV), oxobis(1-phenyl-1,3-butanedionate)vanadium(IV), vanadium(V) oxytriisopropoxide, ammonium metavanadate, sodium metavanadate, vanadium(V) oxide, vanadium(IV) oxide, and vanadyl(IV) sulfate. In view of properties such as the solubility in solvent, preferred are vanadyl(IV) acetylacetonate, and bis(maltolato)oxovanadium(IV), more preferably vanadyl(IV) acetylacetonate.

In view of curability, the content of vanadium compounds is preferably 0.005 to 1 parts by mass relative to total 100 parts by mass of the polymerizable monomer (B) in a dental composition of the present invention.

Preferably, the copper compounds are, for example, compounds that are soluble in radical polymerizable monomers.

Examples of the copper compounds include copper carboxylates, copper β-diketones, copper β-ketoesters, copper alkoxides, copper dithiocarbamates, and salts of copper and inorganic acids.

Examples of copper carboxylates include copper acetate, copper isobutyrate, copper gluconate, copper citrate, copper phthalate, copper tartarate, copper oleate, copper octylate, copper octenate, copper naphthenate, copper methacrylate, and copper 4-cyclohexylbutyrate.

Examples of copper β-diketones include copper acetylacetonate, copper trifluoroacetylacetonate, copper hexafluoroacetylacetonate, copper 2,2,6,6-tetramethyl-3,5-heptanedionate, and copper benzoylacetonate.

Examples of copper β-ketoesters include copper ethyl acetoacetate.

Examples of copper alkoxides include copper methoxide, copper ethoxide, copper isopropoxide, copper 2-(2-butoxyethoxy)ethoxide, and copper 2-(2-methoxyethoxy)ethoxide.

Examples of copper dithiocarbamates include copper dimethyl dithiocarbamate.

Examples of salts of copper and inorganic acids include copper nitrate, and copper chloride.

In view of solubility and reactivity to polymerizable monomer (B), preferred are copper carboxylates, copper β-diketones, and copper β-ketoesters, more preferably copper acetate, and copper acetylacetonate.

In view of curability, the content of copper compounds is preferably 0.000005 to 1 parts by mass, more preferably 0.00001 to 0.5 parts by mass, even more preferably 0.0001 to 0.1 parts by mass relative to total 100 parts by mass of the polymerizable monomer (B) in a dental composition of the present invention.

Examples of other period 4 transition metal compounds include scandium isopropoxide, iron(III) ethoxide, titanium ethoxide, titanium ethoxide, titanium isopropoxide, titanium butoxide, titanium hydroxide, and titanium fluoride.

In view of enhanced polymerization accelerating effect, preferred among the period 4 transition metal compounds are vanadyl(IV) acetylacetonate, and bis(maltolato)oxovanadium(IV), more preferably vanadyl(IV) acetylacetonate.

Examples of transition metal compounds outside of period 4 include strontium carbonate, strontium hydroxide, strontium ethoxide, tin(II) methoxide, indium ethoxide, actinium ethoxide, yttrium isopropoxide, lanthanum methoxide, lanthanum ethoxide, lanthanum isopropoxide, lanthanum butoxide, lanthanum hydroxide, lanthanum carbonate, lanthanum fluoride, cerium isopropoxide, praseodymium isopropoxide, promethium isopropoxide, neodymium isopropoxide, samarium isopropoxide, europium isopropoxide, gadolinium isopropoxide, terbium ethoxide, terbium methoxide, dysprosium isopropoxide, holmium isopropoxide, erbium isopropoxide, thulium isopropoxide, ytterbium isopropoxide, zirconium ethoxide, zirconium isopropoxide, zirconium butoxide, tungsten(IV) methoxide, tungsten(IV) isopropoxide, and tungsten(IV) butoxide.

Examples of thiourea compounds include thiourea, methylthiourea, ethylthiourea, ethylenethiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, tetracyclohexylthiourea, 1-(2-pyridyl)-2-thiourea, (6-methyl-pyridin-2-yl)thiourea, and 4,4-dimethylethylenethiourea.

In view of properties such as the solubility in solvent and storage stability, preferred are 1-(2-pyridyl)-2-thiourea, (6-methyl-pyridin-2-yl)thiourea, and 4,4-dimethylethylenethiourea.

Examples of tin compounds include di-n-butyltin dimaleate, di-n-octyltin dimaleate, di-n-octyltin dilaurate, and di-n-butyltin dilaurate. Particularly preferred tin compounds are di-n-octyltin dilaurate and di-n-butyltin dilaurate.

Examples of aromatic sulfinic acids and salts thereof include benzenesulfinic acid, p-toluenesulfinic acid, o-toluenesulfinic acid, ethylbenzenesulfinic acid, decylbenzenesulfinic acid, dodecylbenzenesulfinic acid, 2,4,6-trimethylbenzenesulfinic acid, 2,4,6-triisopropylbenzenesulfinic acid, chlorobenzenesulfinic acid, naphthalenesulfinic acid, and lithium salts, sodium salts, potassium salts, rubidium salts, cesium salts, magnesium salts, calcium salts, strontium salts, iron salts, zinc salts, ammonium salts, tetramethylammonium salts, and tetraethylammonium salts of these. In view of polymerizability and storage stability of the composition, preferred are 2,4,6-trimethylbenzenesulfinic acid, and lithium salts, sodium salts, potassium salts, magnesium salts, and calcium salts of 2,4,6-triisopropylbenzenesulfinic acid, more preferably lithium salts, sodium salts, potassium salts, magnesium salts, and calcium salts of 2,4,6-triisopropylbenzenesulfinic acid.

Preferably, the aromatic sulfinic acids and salts thereof are at least partially dispersed in powder form within the composition. With the aromatic sulfinic acids and salts thereof being dispersed in powder form, it is easier to provide a longer working time margin for a dental composition of the present invention, and, when the composition is applied to moist materials such as the tooth structure, the aromatic sulfinic acids and salts thereof dissolve in the water on the surface of the moist material, making it possible to more greatly enhance polymerization both at the bonding interface and within the resin-impregnated layer. When the aromatic sulfinic acids and salts thereof are dispersed as powder, it is preferable that the aromatic sulfinic acids and salts thereof have a solubility in water of 1 mg/100 mL or more at ordinary temperature (25°C). A solubility of 1 mg/100 mL or more facilitates sufficient dissolution of aromatic sulfinic acids and salts thereof in the water on moist materials at the bonding interface in applications where a dental composition of the present invention is applied to moist materials, facilitating the effect of dispersing as powder. In view of resistance to settling, the aromatic sulfinic acids and salts thereof have an average particle diameter of preferably 500 µm or less, more preferably 100 µm or less, even more preferably 50 µm or less. In view of preventing excessive increase in the specific surface area of the powder and facilitating in maintaining good ease of handling for the dental composition, the average particle diameter is preferably 0.01 µm or more. That is, when dispersed as powder, the aromatic sulfinic acids and salts thereof have an average particle diameter ranging from preferably 0.01 to 500 µm, more preferably from 0.01 to 100 µm. The average particle diameter can be measured in the same manner as the average particle diameter of the filler (E) described above.

The shape of the aromatic sulfinic acids and salts thereof dispersed as powder is not particularly limited, and may be any of various shapes such as spherical, needle-shape, plate-like, and crushed-shape. Fine powders of aromatic sulfinic acid salts can be prepared using known methods such as pulverization and freeze drying.

In view of working time margin and adhesive properties, the content of aromatic sulfinic acids and salts thereof is preferably 0.1 to 5 parts by mass, more preferably 0.2 to 4 parts by mass, even more preferably 0.5 to 3 parts by mass relative to total 100 parts by mass of the polymerizable monomer (B) in an adhesive composition of the present invention. A content of less than 0.1 parts by mass may cause a decrease in adhesive properties, whereas a content of more than 5 parts by mass may result in shorter working time margins.

Examples of bromides include zinc bromide, potassium bromide, sodium bromide, calcium bromide, indium bromide, ammonium bromide, and tetrabutylammonium bromide. Particularly preferred are zinc bromide, ammonium bromide, and tetrabutylammonium bromide.

Examples of borate compounds include arylborate compounds having 1 to 4 aryl groups per molecule (for example, such as tetraphenylboron, and tetrakis(p-chlorophenyl)boron), and salts of these.

Examples of barbituric acid compounds include barbituric acid, 5-butyl barbituric acid, 1,3,5-trimethyl barbituric acid, 1-cyclohexyl-5-ethyl barbituric acid, 1-benzyl-5-phenyl barbituric acid, and salts of these.

Examples of ascorbic acid compounds include salts, esters, and ethers of ascorbic acid. Preferred are salts and esters of ascorbic acid.

Examples of salts of ascorbic acid include sodium L-ascorbate, calcium L-ascorbate, potassium ascorbate, and stereoisomers of these (for example, such as sodium isoascorbate). Preferred is sodium L-ascorbate.

Examples of esters of ascorbic acid include compounds formed by reacting one or more hydroxyl groups of ascorbic acid with a carboxylic acid. Preferred examples of the carboxylic acid include C6 to C30 saturated or unsaturated fatty acids, such as caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid, and docosahexaenoic acid. The fatty acids have preferably 10 to 28 carbon atoms, more preferably 12 to 26 carbon atoms, even more preferably 14 to 24 carbon atoms. Particularly preferred for use are esters of stearic acid and ascorbic acid, and esters of palmitic acid and ascorbic acid (ascorbyl palmitate).

Examples of ethers of ascorbic acid include ethyl ascorbic ether, and cetyl ascorbic ether.

Examples of sulfur-containing reducing inorganic compounds include sulfites, bisulfites, pyrosulfites, thiosulfates, thionates, and dithionites. Preferred are sulfites and bisulfites.

Specific examples of sulfur-containing reducing inorganic compounds include sodium sulfite, potassium sulfite, calcium sulfite, ammonium sulfite, sodium bisulfite, and potassium bisulfite.

Preferably, the sulfur-containing reducing inorganic compound is at least partially dispersed in powder form within the composition. With the sulfur-containing reducing inorganic compound being dispersed in powder form, it is possible to provide a longer working time margin for a dental composition of the present invention, and, when the composition is applied to the tooth structure, the sulfur-containing reducing inorganic compound dissolves in the water on the surface of the tooth structure, making it possible to more greatly enhance polymerization both at the bonding interface and within the resin-impregnated layer. When the sulfur-containing reducing inorganic compound is dispersed as powder, it is preferable that the sulfur-containing reducing inorganic compound have a solubility in water of 1 mg/100 mL or more at ordinary temperature (25°C). A solubility of 1 mg/100 mL or more facilitates sufficient dissolution of the sulfur-containing reducing inorganic compound in the water on tooth structure at the bonding interface in applications where a dental composition of the present invention is applied to tooth structure, facilitating the effect of dispersing as powder. In view of resistance to settling, the sulfur-containing reducing inorganic compound has an average particle diameter of preferably 500 µm or less, more preferably 100 µm or less, even more preferably 50 µm or less. In view of preventing excessive increase in the specific surface area of the powder and facilitating in maintaining good ease of handling for the dental composition, the average particle diameter is preferably 0.01 µm or more. That is, when dispersed as powder, the sulfur-containing reducing inorganic compound has an average particle diameter ranging from preferably 0.01 to 500 µm, more preferably from 0.01 to 100 µm. The average particle diameter can be measured in the same manner as the average particle diameter of filler (E).

The shape of the sulfur-containing reducing inorganic compound dispersed as powder is not particularly limited, and may be any of various shapes such as spherical, needle-shape, plate-like, and crushed-shape. Fine powders of sulfur-containing reducing inorganic compounds can be prepared using known methods such as pulverization and freeze drying.

A dental composition of the present invention may comprise a fluoride ion-releasing substance to impart acid resistance to tooth structure. The fluoride ion-releasing substance may be used alone, or two or more thereof may be used in combination.

Examples of the fluoride ion-releasing substance include fluoride ion-releasing polymers such as copolymers of methyl methacrylate and methacryloyl fluoride; fluoride ion-releasing substances such as cetylamine hydrofluoride; and inorganic fillers such as fluoroaluminosilicate glass, sodium fluoride, potassium fluoride, sodium monofluorophosphate, lithium fluoride, and ytterbium fluoride.

A dental composition of the present invention may comprise additives such as a cross-linking agent, a silanol condensation catalyst, a stabilizer (polymerization inhibitor), a colorant (dye, pigment), and a fluorescent agent. The additives may be used alone, or two or more thereof may be used in combination.

The cross-linking agent and the silanol condensation catalyst may be those described in WO2019/004391.

Examples of the polymerization inhibitor include hydroquinone, hydroquinone monomethyl ether, dibutyl hydroquinone, dibutyl hydroquinone monomethyl ether, t-butyl catechol, 2-t-butyl-4,6-dimethylphenol, 2,6-di-t-butyl-4-methylphenol, and 2,6-di-t-butylphenol.

It is also possible to comprise anti-microbial substances such as cetylpyridinium chloride, benzalkonium chloride, (meth)acryloyloxydodecylpyridinium bromide, (meth)acryloyloxyhexadecylpyridinium chloride, (meth)acryloyloxydecylammonium chloride, and triclosan. The anti-microbial substances may be used alone, or two or more thereof may be used in combination.

A dental composition of the present invention may be a one-pack or two-pack type dental composition, without particular restrictions.

In view of handling properties, a certain preferred embodiment of a dental composition of the present invention is, for example, a one-pack type dental composition.

A dental composition of the present invention may comprise a solvent (water, organic solvent).

In a certain preferred embodiment, the content of the solvent (water, organic solvent) in a dental composition of the present invention is preferably 0.1 mass% or more, more preferably 1.0 mass% or more, even more preferably 5.0 mass% or more. The solvent content (water, organic solvent) is preferably 50 mass% or less, more preferably 45 mass% or less, even more preferably 40 mass% or less.

When a dental composition of the present invention is used in applications such as dental composite resins, it is preferable not to comprise a solvent (water, organic solvent).

In another certain preferred embodiment, the content of the solvent (water, organic solvent) in a dental composition of the present invention is preferably less than 5.0 mass%, more preferably less than 1.0 mass%, even more preferably less than 0.5 mass%, particularly preferably less than 0.1 mass%, most preferably less than 0.01 mass%.

A dental composition of the present invention can be produced, for example, by mixing components other than the powder components (e.g., filler (E)) to obtain a solution, and subsequently adding the powder components.

The present invention encompasses embodiments combining all or part of the foregoing features in various forms, provided that the present invention can exhibit its effects with such combinations within the technical idea of the present invention.

### EXAMPLES

The present invention is described below in greater detail through Examples. It is to be noted, however, that the present invention is in no way limited by the following Examples, and various modifications may be made by a person with ordinary skill in the art within the technical idea of the present invention. The following abbreviations and symbols are used.

### [Ultraviolet Absorber (A)]

T1: bisethylhexyloxyphenol methoxyphenyl triazine
T2: 2-[4,6-bis(biphenyl-4-yl)-1,3,5-triazin-2-yl]-5-[(2-ethylhexyl)oxy]phenol [0145] [Ultraviolet Absorbers Other Than Ultraviolet Absorber (A)]
T3: 2,4,6-tris[4-(hexyloxy)-2-hydroxy-3-methylphenyl]-1,3,5-triazine
T4: 2,4-bis(2,4-dimethylphenyl)-6-(2-hydroxy-4-n-octyloxyphenyl)-1,3,5-triazine
T5: 2-(2-hydroxy-4-methoxyphenyl)-4,6-diphenyl-1,3,5-triazine
T6: 2,4,6-tri([1,1'-biphenyl]-4-yl)-1,3,5-triazine
B1: 2-(3-tert-butyl-2-hydroxy-5-methylphenyl)-5-chloro-2H-benzotriazole
B2: 2-(2H-benzotriazol-2-yl)-4-methyl-6-dodecylphenol
O1: 2,2'-(1,4-phenylene)bis(4H-3,1-benzooxazin-4-one)
O2: 2-ethylhexyl 4-methoxycinnamate

### [Polymerizable Monomer Having Acidic Group (B-1)]

MDP: 10-methacryloyloxydecyl dihydrogen phosphate

### [Polymerizable Monomer Having no Acidic Group (B-2)]

UDMA: 2,2,4-trimethylhexamethylenebis(2-carbamoyloxyethyl) dimethacrylate
HEMA: 2-hydroxyethyl methacrylate
Bis-GMA: 2,2-bis[4-(2-hydroxy-3-methacryloyloxypropoxy)phenyl]propane
3G: triethylene glycol dimethacrylate
NPG: neopentyl glycol dimethacrylate
D-2.6E: 2,2-bis(4-methacryloyloxypolyethoxyphenyl)propane (with an average of 2.6 moles of ethoxy group added)
DEAA: N,N-diethylacrylamide
MAEA: N-methacryloyloxyethylacrylamide
THF-MA: tetrahydrofurfuryl methacrylate
UN7600: urethane acrylate (manufactured by Negami Chemical Industrial Co., Ltd.; weight-average molecular weight (Mw): 11,500; number of polymerizable groups: 2)

### [Photopolymerization Initiator (C)]

CQ: dl-camphorquinone
BAPO: bis(2,4,6-trimethylbenzoyl)phenylphosphine oxide
BAPO-ONa: sodium bis(2,4,6-trimethylbenzoyl)phosphinate
TMDPO: 2,4,6-trimethylbenzoyldiphenylphosphine oxide

### [Polymerization Accelerator (D)]

DABE: ethyl 4-(N,N-dimethylamino)benzoate
DEPT: N,N-bis(2-hydroxyethyl)-p-toluidine

### [Filler (E)]

R972: silica dimethyl silylate (Aerosil^{®} R972 manufactured by Nippon Aerosil Co., Ltd.; average primary particle diameter: 16 nm)
Ar380: hydrophilic fumed silica (Aerosil^{®} 380 manufactured by Nippon Aerosil Co., Ltd.; average primary particle diameter: 7 nm)
Filler 1: silica (Silica Micro Bead P-500KN manufactured by JGC C & C; average particle diameter: 2 µm)
Filler 2: A three-neck flask was charged with 100 parts by mass of barium glass (GM27884 manufactured by Schott under this trade name; average particle diameter of primary particles: 0.18 µm), 11 parts by mass of 3-methacryloyloxypropyltrimethoxysilane, and 500 mL of a 0.5% acetic acid aqueous solution. These were then stirred at room temperature for 2 hours under ultrasonic dispersion. After freeze drying to remove water, the resulting material was subjected to heat treatment at 90°C for 3 hours to yield a surface-treated BaO filler. This was used as filler 2.
Filler 3: A silica stone powder (Hi-Silica manufactured by Nitchitsu Co., Ltd. under this trade name) was pulverized with a dry ball mill (10 mm-diameter alumina balls) to obtain a pulverized silica stone powder. The pulverized silica stone powder had an average particle diameter of 2.2 µm as measured by volume with a laser diffraction particle size distribution analyzer (Model SALD-2300, manufactured by Shimadzu Corporation). Subsequently, 100 g of this pulverized silica stone powder was charged into a three-neck flask along with 4 g of γ-methacryloyloxypropyltrimethoxysilane, and 200 mL of an acetic acid aqueous solution. These were then stirred at room temperature for 2 hours under ultrasonic dispersion. After freeze drying to remove water, the mixture was subjected to heat treatment at 90°C for 3 hours to yield a filler. This was used as filler 3.
Filler 4: A three-neck flask was charged with 100 g of Ar130 (hydrophilic fumed silica; ultrafine particulate silica Aerosil^{®} 130 manufactured by Nippon Aerosil Co., Ltd. under this trade name; average particle diameter: 16 nm), 30 g of 3-methacryloyloxypropyltrimethoxysilane, and 200 mL of a 0.3 mass% acetic acid aqueous solution. These were then stirred at room temperature for 2 hours under ultrasonic dispersion. After freeze drying to remove water, the mixture was subjected to heat treatment at 90°C for 3 hours to yield a surface-treated SiO₂ silica. This was used as filler 4.
Filler 5: A commercially available surface-treated YbF₃ with SiO₂ coating (SG-YBF100WSCMP10 manufactured by Sukgyung AT; average particle diameter of primary particles: 110 nm, average particle diameter of secondary particles: 1.2 µm; refractive index: 1.53) was used as filler 5.

### [Others]

BHT: 2,6-di-t-butyl-4-methylphenol (polymerization inhibitor)

### [Examples 1-1 to 1-8, 2-1 to 2-8, 3-1 to 3-6, and 4-1 to 4-5, and Comparative Examples 1-1 to 1-9, 2-1 to 2-9, 3-1 and 3-2, and 4-1 and 4-2]

The mixtures having the formulations shown in Tables 2 to 5 were used for evaluations as dental compositions.

The dental compositions were prepared specifically as follows. For the dental bonding materials (Tables 2 and 3), components other than the filler and the solvent (water, organic solvent) were mixed first, before adding and mixing the filler and subsequently the solvent (water, organic solvent). The dental composite resins (Tables 4 and 5) were produced by adding and mixing the filler after mixing components other than the filler.

The dental compositions produced were subjected to testing following the methods described below. The results are presented in Tables 2 to 5.

The ultraviolet absorbers were measured to determine the solubility in a hydrophilic polymerizable monomer and the maximum absorption wavelength, following the methods described below. The results are presented in Table 1.

### [Measurement of Maximum Absorption Wavelength]

Using ethyl acetate as the solvent, the ultraviolet absorber was dissolved to prepare a solution at a concentration of 3 × 10⁻⁵ mol/L. This solution was then measured for maximum absorption wavelength over a wavelength range of 250 nm to 500 nm, using a spectrophotometer (UH5200, manufactured by Hitachi High-Tech Corporation under this trade name) with a quartz cell having an optical path length of 10 mm (arithmetic mean value for n = 2).

### [Solubility in Hydrophilic Polymerizable Monomer]

The solubility of the ultraviolet absorber in a hydrophilic polymerizable monomer was evaluated as follows.

Using 2-hydroxyethyl methacrylate (HEMA) as a hydrophilic polymerizable monomer, 0.05 parts by mass of the ultraviolet absorber was added to 100 parts by mass of 2-hydroxyethyl methacrylate, and the mixture was stirred at 25°C and 1,000 rpm using a stirrer. After 1 hour of stirring, whether the ultraviolet absorber had dissolved was visually confirmed. If dissolution was observed, the ultraviolet absorber was continuously added in increments of 0.01 parts by mass, each followed by 1 hour of stirring, and the procedure was repeated until the ultraviolet absorber no longer dissolved. If no dissolution was observed, stirring was continued for 24 hours, and the presence or absence of dissolution was visually confirmed. If dissolution was observed, an additional 0.01 parts by mass was added, and the solution was visually inspected after 24 hours of stirring to determine whether the ultraviolet absorber had dissolved. This procedure was repeated until the ultraviolet absorber no longer dissolved. The solubility in the hydrophilic polymerizable monomer was determined as the amount, in parts by mass, at which the ultraviolet absorber remained dissolved (arithmetic mean value for n = 2).

### [Solubility]

For each Example and Comparative Example, a dental composition was prepared without the solvent (water and ethanol) and the ultraviolet absorber (ultraviolet absorber (A), and ultraviolet absorbers other than ultraviolet absorber (A)). The ultraviolet absorber was then added in the amount specified for each Example and Comparative Example, and the mixture was stirred for 24 hours at 25°C and 1,000 rpm using a stirrer. Taking Example 1-1 as an example, 0.26 parts by mass of T1 was added as ultraviolet absorber (A) to the dental composition prepared in 99.74 parts by mass.

The mixture was visually inspected to determine whether the ultraviolet absorber had dissolved, and rated "A" when the ultraviolet absorber had completely dissolved, and "B" when the ultraviolet absorber remained undissolved.

### [Unpolymerized Thickness]

The unpolymerized thickness was determined as a measure of photocurability, as follows.

A polyester film was laid on a glass slide, and a 1 mm-thick Teflon^{®} mold with a hole (Ø = 5 mm) was placed on the film. The dental composition prepared above was then filled into the mold, and its mass was measured as (X) [g].

The dental composition was irradiated with light for 10 seconds, using a dental polymerization photoirradiator PenCure 2000 (manufactured by J. Morita Corp. under this trade name). Subsequently, the uncured portion was removed by wiping it off with a JK Wiper^{®} (manufactured by Nippon Paper Crecia Co., Ltd.), and the remaining mass was measured as (Y) [g]. The unpolymerized thickness was calculated using the following equation (arithmetic mean value for n = 5). 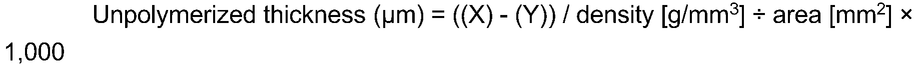 Unpolymerized thickness (µm) = ((X) - (Y)) / density [g/mm3] ÷ area [mm2] × 1,000

### [Stability to 8,000 lux Ambient Light]

Using an LED-equipped operating light (Luna Vue EL, manufactured by J. Morita Corp. under this trade name), the height and output were adjusted to achieve an illuminance of 8,000 lux.

For the dental bonding material, a single drop of a sample (e.g., approximately 0.02 g of the composition of Example 2-1) was applied to a dental mixing dish (manufactured by Kuraray Noritake Dental Inc.; part number: #912 (TB)). The sample was exposed to light while measuring the elapsed time (1 second to a maximum of 100 seconds). At a suitable time point, the mixing dish with the dispensed sample was taken out of the irradiation zone, and the sample was immediately examined using a fine brush to check for the presence or absence of solidification or gelation through visual inspection, in order to confirm whether the sample maintained physical uniformity. The first occurrence of solidification or gelation was identified, and the time one second before this occurrence was determined as the period of maintained uniformity. The working time margin (unit: seconds) was calculated based on this result.

For the dental composite resin, about 0.05 g of a sample was weighed out on a glass slide. The sample was exposed to light while measuring the elapsed time (1 second to a maximum of 100 seconds). At a suitable time point, the glass slide with the weighed sample was taken out of the irradiation zone, and the sample was immediately examined by pressing it from above with another glass slide and visually checking for the presence or absence of solidification or gelation, in order to confirm whether the sample maintained physical uniformity. The first occurrence of solidification or gelation was identified, and the time one second before this occurrence was determined as the period of maintained uniformity. The working time margin (unit: seconds) was calculated based on this result.

### [Stability to 27,000 lux Ambient Light]

The working time margin (unit: seconds) was determined using the same method described in [Stability to 8,000 lux Ambient Light], except that the illuminance was adjusted to 27,000 lux.

**[Table 1]**

| | | Presence or absence of triazine skeleton | Amount dissolved relative to 100 parts by mass of 2-hydroxyethyl methacrylate | Maximum absorption wavelength (nm) |
|---|---|---|---|---|
| Ultraviolet absorber (A) | T1 | Present | 0.35 parts by mass | 350 to 360 |
| | T2 | Present | 0.15 parts by mass | 315 to 325 |
| Ultraviolet absorbers other than ultraviolet absorber (A) | B1 | Absent | 0.65 parts by mass | 350 to 360 |
| | B2 | Absent | 1 part or more by mass | 345 to 355 |
| | T3 | Present | 0.05 parts or less by mass | 355 to 365 |
| | T4 | Present | 0.15 parts by mass | 285 to 295 |
| | T5 | Present | 0.1 parts by mass | 335 to 345 |
| | T6 | Present | 0.05 parts or less by mass | 310 to 320 |
| | O1 | Absent | 0.05 parts or less by mass | 340 to 350 |
| | O2 | Absent | 1 part or more by mass | 310 to 320 |

**[Table 2]**

| Components (mass%) | | Ex. 1-1 | Ex. 1-2 | Ex. 1-3 | Ex. 1-4 | Ex. 1-5 | Ex. 1-6 | Ex. 1-7 | Ex. 1-8 |
|---|---|---|---|---|---|---|---|---|---|
| Polymerizable monomer having acidic group (B-1) | MDP | 4.50 | 4.40 | 4.40 | 4.50 | | 4.80 | 4.50 | 4.50 |
| Polymerizable monomer having no acidic group (B-2) | Bis-GMA | 35.00 | 35.00 | 35.00 | 35.00 | 37.00 | 38.00 | 35.00 | 35.00 |
| | HEMA | 30.54 | 30.55 | 30.70 | 30.54 | 31.98 | 33.57 | 30.54 | |
| | DEAA | | | | | | | | 30.54 |
| | NPG | 17.50 | 17.50 | 17.55 | 17.50 | 18.00 | 19.10 | 17.50 | 17.50 |
| Photopolymerization initiator (C) | CQ | 0.70 | 0.70 | 0.70 | 0.70 | 0.70 | 0.80 | 0.70 | 0.70 |
| | BAPO | 0.90 | 0.90 | 0.90 | 0.90 | 0.90 | 1.00 | 0.90 | 0.90 |
| Ultraviolet absorber (A) | T1 | 0.26 | 0.35 | 0.18 | | 0.27 | 0.29 | 0.13 | 0.26 |
| | T2 | | | | 0.26 | | | 0.13 | |
| Ultraviolet absorbers other than ultraviolet absorber (A) | B1 | | | | | | | | |
| | B2 | | | | | | | | |
| | T3 | | | | | | | | |
| | T4 | | | | | | | | |
| | T5 | | | | | | | | |
| | T6 | | | | | | | | |
| | O1 | | | | | | | | |
| | O2 | | | | | | | | |
| Polymerization accelerator (D) | DABE | 1.75 | 1.75 | 1.75 | 1.75 | 1.80 | 1.90 | 1.75 | 1.75 |
| | DEPT | 0.50 | 0.50 | 0.48 | 0.50 | 0.45 | 0.50 | 0.50 | 0.50 |
| Filler (E) | R972 | 7.00 | 7.00 | 7.00 | 7.00 | 7.50 | | 7.00 | 7.00 |
| | Ar380 | 1.30 | 1.30 | 1.30 | 1.30 | 1.35 | | 1.30 | 1.30 |
| Other components | BHT | 0.04 | 0.04 | 0.04 | 0.04 | 0.05 | 0.05 | 0.04 | 0.04 |
| Solubility | | A | A | A | A | A | A | A | A |
| Unpolymerized thickness (µm) | | 5.4 | 6.4 | 4.9 | 5.9 | 5.3 | 6.5 | 6.1 | 5.8 |
| Stability to 8,000 lux ambient light (s) | | 23 | 23 | 23 | 23 | 23 | 23 | 23 | 23 |
| Stability to 27,000 lux ambient light (s) | | 8 | 8 | 8 | 8 | 8 | 8 | 8 | 8 |

**[Table 2] Continued**

| Components (mass%) | | Com. Ex. 1-1 | Com. Ex. 1-2 | Com. Ex. 1-3 | Com. Ex. 1-4 | Com. Ex. 1-5 | Com. Ex. 1-6 | Com. Ex. 1-7 | Com. Ex. 1-8 | Com. Ex. 1-9 |
|---|---|---|---|---|---|---|---|---|---|---|
| Polymerizable monomer having acidic group (B-1) | MDP | 4.50 | 4.50 | 4.50 | 4.50 | 4.50 | 4.50 | 4.50 | 4.50 | 4.50 |
| Polymerizable monomer having no acidic group (B-2) | Bis-GMA | 35.00 | 35.00 | 35.00 | 35.00 | 35.00 | 35.00 | 35.00 | 35.00 | 35.00 |
| | HEMA | 30.54 | 30.54 | 30.54 | 30.54 | 30.54 | 30.54 | 30.54 | 30.54 | 30.54 |
| | DEAA | | | | | | | | | |
| | NPG | 17.50 | 17.50 | 17.50 | 17.50 | 17.50 | 17.50 | 17.50 | 17.50 | 17.50 |
| Photopolymerization initiator (C) | CQ | 0.70 | 0.70 | 0.70 | 0.70 | 0.70 | 0.70 | 0.70 | 0.70 | 0.70 |
| | BAPO | 0.90 | 0.90 | 0.90 | 0.90 | 0.90 | 0.90 | 0.90 | 0.90 | 0.90 |
| Ultraviolet absorber (A) | T1 | | | | | | | | | |
| | T2 | | | | | | | | | |
| Ultraviolet absorbers other than ultraviolet absorber (A) | B1 | 0.26 | | | | | | | | 0.13 |
| | B2 | | 0.26 | | | | | | | |
| | T3 | | | 0.26 | | | | | | |
| | T4 | | | | 0.26 | | | | | 0.13 |
| | T5 | | | | | 0.26 | | | | |
| | T6 | | | | | | 0.26 | | | |
| | O1 | | | | | | | 0.26 | | |
| | O2 | | | | | | | | 0.26 | |
| Polymerization accelerator (D) | DABE | 1.75 | 1.75 | 1.75 | 1.75 | 1.75 | 1.75 | 1.75 | 1.75 | 1.75 |
| | DEPT | 0.50 | 0.50 | 0.50 | 0.50 | 0.50 | 0.50 | 0.50 | 0.50 | 0.50 |
| Filler (E) | R972 | 7.00 | 7.00 | 7.00 | 7.00 | 7.00 | 7.00 | 7.00 | 7.00 | 7.00 |
| | Ar380 | 1.30 | 1.30 | 1.30 | 1.30 | 1.30 | 1.30 | 1.30 | 1.30 | 1.30 |
| Other components | BHT | 0.04 | 0.04 | 0.04 | 0.04 | 0.04 | 0.04 | 0.04 | 0.04 | 0.04 |
| Solubility | | A | A | B | A | A | B | B | A | A |
| Unpolymerized thickness (µm) | | 7.9 | 7.6 | | 5.7 | 7.3 | | | 6.3 | 7.0 |
| Stability to 8,000 lux ambient light (s) | | 22 | 22 | | 22 | 22 | | | 23 | 23 |
| Stability to 27,000 lux ambient light (s) | | 5 | 5 | | 5 | 5 | | | 5 | 5 |

**[Table 3]**

| Components (mass%) | | Ex. 2-1 | Ex. 2-2 | Ex. 2-3 | Ex. 2-4 | Ex. 2-5 | Ex. 2-6 | Ex. 2-7 | Ex. 2-8 |
|---|---|---|---|---|---|---|---|---|---|
| Polymerizable monomer having acidic group (B-1) | MDP | 8.50 | 8.50 | 8.50 | 8.50 | | 8.90 | 8.50 | 8.50 |
| Polymerizable monomer having no acidic group (B-2) | Bis-GMA | 25.50 | 25.45 | 25.50 | 25.50 | 27.80 | 26.60 | 25.50 | 25.50 |
| | HEMA | 8.34 | 8.47 | 8.47 | 8.34 | 9.30 | 8.90 | 8.34 | |
| | DEAA | 8.50 | 8.50 | 8.50 | 8.50 | 9.30 | 8.90 | 8.50 | 16.84 |
| | MAEA | 12.75 | 12.75 | 12.75 | 12.75 | 13.90 | 13.30 | 12.75 | 12.75 |
| Photopolymerization initiator (C) | CQ | 1.70 | 1.70 | 1.70 | 1.70 | 1.85 | 1.80 | 1.70 | 1.70 |
| | BAPO | 0.65 | 0.65 | 0.64 | 0.65 | 0.70 | 0.65 | 0.65 | 0.65 |
| Ultraviolet absorber (A) | T1 | 0.13 | 0.15 | 0.11 | | 0.14 | 0.13 | 0.06 | 0.13 |
| | T2 | | | | 0.13 | | | 0.06 | |
| Ultraviolet absorbers other than ultraviolet absorber (A) | B1 | | | | | | | | |
| | B2 | | | | | | | | |
| | T3 | | | | | | | | |
| | T4 | | | | | | | | |
| | T5 | | | | | | | | |
| | T6 | | | | | | | | |
| | O1 | | | | | | | | |
| | O2 | | | | | | | | |
| Polymerization accelerator (D) | DABE | 1.70 | 1.70 | 1.70 | 1.70 | 1.85 | 1.75 | 1.70 | 1.70 |
| | DEPT | 0.75 | 0.75 | 0.75 | 0.75 | 0.80 | 0.80 | 0.75 | 0.75 |
| Filler (E) | R972 | 4.25 | 4.25 | 4.24 | 4.25 | 4.65 | | 4.25 | 4.25 |
| Other components | BHT | 0.04 | 0.04 | 0.04 | 0.04 | 0.05 | 0.04 | 0.04 | 0.04 |
| Solvent | Water | 14.50 | 14.40 | 14.40 | 14.50 | 15.76 | 14.93 | 14.50 | 14.50 |
| | Ethanol | 12.70 | 12.70 | 12.70 | 12.70 | 13.90 | 13.30 | 12.70 | 12.70 |
| Solubility | | A | A | A | A | A | A | A | A |
| Unpolymerized thickness (µm) | | 6.6 | 5.9 | 6.8 | 5.8 | 6.4 | 6.2 | 6.1 | 6.8 |
| Stability to 8,000 lux ambient light (s) | | 40 | 41 | 40 | 40 | 40 | 40 | 40 | 40 |
| Stability to 27,000 lux ambient light (s) | | 17 | 17 | 17 | 17 | 17 | 17 | 17 | 17 |

**[Table 3] Continued**

| Components (mass%) | | Com. Ex. 2-1 | Com. Ex. 2-2 | Com. Ex. 2-3 | Com. Ex. 2-4 | Com. Ex. 2-5 | Com. Ex. 2-6 | Com. Ex. 2-7 | Com. Ex. 2-8 | Com. Ex. 2-9 |
|---|---|---|---|---|---|---|---|---|---|---|
| Polymerizable monomer having acidic group (B-1) | MDP | 8.50 | 8.50 | 8.50 | 8.50 | 8.50 | 8.50 | 8.50 | 8.50 | 8.50 |
| Polymerizable monomer having no acidic group (B-2) | Bis-GMA | 25.50 | 25.50 | 25.50 | 25.50 | 25.50 | 25.50 | 25.50 | 25.50 | 25.50 |
| | HEMA | 8.34 | 8.34 | 8.34 | 8.34 | 8.34 | 8.34 | 8.34 | 8.34 | 8.34 |
| | DEAA | 8.50 | 8.50 | 8.50 | 8.50 | 8.50 | 8.50 | 8.50 | 8.50 | 8.50 |
| | MAEA | 12.75 | 12.75 | 12.75 | 12.75 | 12.75 | 12.75 | 12.75 | 12.75 | 12.75 |
| Photopolymerization initiator (C) | CQ | 1.70 | 1.70 | 1.70 | 1.70 | 1.70 | 1.70 | 1.70 | 1.70 | 1.70 |
| | BAPO | 0.65 | 0.65 | 0.65 | 0.65 | 0.65 | 0.65 | 0.65 | 0.65 | 0.65 |
| Ultraviolet absorber (A) | T1 | | | | | | | | | |
| | T2 | | | | | | | | | |
| Ultraviolet absorbers other than ultraviolet absorber (A) | B1 | 0.13 | | | | | | | | 0.06 |
| | B2 | | 0.13 | | | | | | | |
| | T3 | | | 0.13 | | | | | | |
| | T4 | | | | 0.13 | | | | | 0.06 |
| | T5 | | | | | 0.13 | | | | |
| | T6 | | | | | | 0.13 | | | |
| | O1 | | | | | | | 0.13 | | |
| | O2 | | | | | | | | 0.13 | |
| Polymerization accelerator (D) | DABE | 1.70 | 1.70 | 1.70 | 1.70 | 1.70 | 1.70 | 1.70 | 1.70 | 1.70 |
| | DEPT | 0.75 | 0.75 | 0.75 | 0.75 | 0.75 | 0.75 | 0.75 | 0.75 | 0.75 |
| Filler (E) | R972 | 4.25 | 4.25 | 4.25 | 4.25 | 4.25 | 4.25 | 4.25 | 4.25 | 4.25 |
| Other components | BHT | 0.04 | 0.04 | 0.04 | 0.04 | 0.04 | 0.04 | 0.04 | 0.04 | 0.04 |
| Solvent | Water | 14.50 | 14.50 | 14.50 | 14.50 | 14.50 | 14.50 | 14.50 | 14.50 | 14.50 |
| | Ethanol | 12.70 | 12.70 | 12.70 | 12.70 | 12.70 | 12.70 | 12.70 | 12.70 | 12.70 |
| Solubility | | A | A | B | A | A | B | B | A | A |
| Unpolymerized thickness (µm) | | 8.5 | 8.1 | | 6.1 | 7.6 | | | 7.2 | 6.6 |
| Stability to 8,000 lux ambient light (s) | | 39 | 39 | | 38 | 39 | | | 38 | 38 |
| Stability to 27,000 lux ambient light (s) | | 12 | 12 | | 12 | 12 | | | 12 | 12 |

**[Table 4]**

| Components (mass%) | | Ex. 3-1 | Ex. 3-2 | Ex. 3-3 | Ex. 3-4 | Ex. 3-5 | Ex. 3-6 | Com. Ex. 3-1 | Com. Ex. 3-2 |
|---|---|---|---|---|---|---|---|---|---|
| Polymerizable monomer having no acidic group (B-2) | 3G | 7.5 | 7.5 | 7.5 | 7.5 | 7.5 | 7.5 | 7.5 | 7.5 |
| | D-2.6E | 17.20 | 17.23 | 17.17 | 17.20 | 17.20 | 17.20 | 17.20 | 17.20 |
| Photopolymerization initiator (C) | CQ | 0.05 | 0.05 | 0.05 | 0.05 | 0.05 | 0.05 | 0.05 | 0.05 |
| | TMDPO | 0.10 | 0.10 | 0.10 | 0.10 | 0.10 | | 0.10 | 0.10 |
| Ultraviolet absorber (A) | T1 | 0.06 | 0.03 | 0.09 | | 0.03 | 0.06 | | |
| | T2 | | | | 0.06 | 0.03 | | | |
| Ultraviolet absorbers other than ultraviolet absorber (A) | B1 | | | | | | | 0.06 | 0.03 |
| | T4 | | | | | | | | 0.03 |
| Polymerization accelerator (D) | DABE | 0.06 | 0.06 | 0.06 | 0.06 | 0.06 | 0.06 | 0.06 | 0.06 |
| Filler (E) | Filler 1 | 5 | 5 | 5 | 5 | 5 | 5 | 5 | 5 |
| | Filler 2 | 70 | 70 | 70 | 70 | 70 | 70 | 70 | 70 |
| Other components | BHT | 0.03 | 0.03 | 0.03 | 0.03 | 0.03 | 0.03 | 0.03 | 0.03 |
| Solubility | | A | A | A | A | A | A | A | A |
| Unpolymerized thickness (µm) | | 9 | 9 | 10 | 10 | 9 | 7 | 9 | 9 |
| Stability to 8,000 lux ambient light (s) | | 75 | 75 | 80 | 80 | 75 | 65 | 70 | 65 |
| Stability to 27,000 lux ambient light (s) | | 21 | 22 | 24 | 22 | 21 | 16 | 13 | 11 |

**[Table 5]**

| Components (mass%) | | Ex. 4-1 | Ex. 4-2 | Ex. 4-3 | Ex. 4-4 | Ex. 4-5 | Com. Ex. 4-1 | Com. Ex. 4-2 |
|---|---|---|---|---|---|---|---|---|
| Polymerizable monomer having acidic group (B-1) | MDP | 1.5 | 1.5 | 1.5 | 1.5 | 1.5 | 1.5 | 1.5 |
| Polymerizable monomer having no acidic group (B-2) | D-2.6E | 9.0 | 9.0 | 9.0 | 9.0 | 9.0 | 9.0 | 9.0 |
| | UDMA | 11.60 | 11.64 | 11.56 | 11.60 | 11.60 | 11.60 | 11.60 |
| | MAEA | 1.5 | 1.5 | 1.5 | 1.5 | 1.5 | 1.5 | 1.5 |
| | THF-MA | 5.0 | 5.0 | 5.0 | 5.0 | 5.0 | 5.0 | 5.0 |
| | UN7600 | 6.0 | 6.0 | 6.0 | 6.0 | 6.0 | 6.0 | 6.0 |
| Photopolymerization initiator (C) | CQ | 0.08 | 0.08 | 0.08 | 0.08 | 0.08 | 0.08 | 0.08 |
| | BAPO-ONa | 0.13 | 0.13 | 0.13 | 0.13 | 0.13 | 0.13 | 0.13 |
| Ultraviolet absorber (A) | T1 | 0.08 | 0.04 | 0.12 | | 0.04 | | |
| | T2 | | | | 0.08 | 0.04 | | |
| Ultraviolet absorbers other than ultraviolet absorber (A) | B1 | | | | | | 0.08 | 0.04 |
| | T4 | | | | | | | 0.04 |
| Polymerization accelerator (D) | DABE | 0.08 | 0.08 | 0.08 | 0.08 | 0.08 | 0.08 | 0.08 |
| Filler (E) | Filler 3 | 50.0 | 50.0 | 50.0 | 50.0 | 50.0 | 50.0 | 50.0 |
| | Filler 4 | 2.0 | 2.0 | 2.0 | 2.0 | 2.0 | 2.0 | 2.0 |
| | Filler 5 | 13.0 | 13.0 | 13.0 | 13.0 | 13.0 | 13.0 | 13.0 |
| Other components | BHT | 0.03 | 0.03 | 0.03 | 0.03 | 0.03 | 0.03 | 0.03 |
| Solubility | | A | A | A | A | A | A | A |
| Unpolymerized thickness (µm) | | 10 | 11 | 7 | 8 | 8 | 11 | 11 |
| Stability to 8,000 lux ambient light (s) | | 50 | 55 | 45 | 50 | 50 | 50 | 45 |
| Stability to 27,000 lux ambient light(s) | | 15 | 14 | 17 | 15 | 15 | 12 | 9 |

As indicated in Tables 2 to 5, the dental compositions of the present invention, irrespective of the Example employed for preparation, exhibited superior ambient light stability under high illuminance compared with Comparative Examples of similar constitutions.

Furthermore, the dental compositions of the present invention, irrespective of the Example employed for preparation, exhibited superior photocurability due to the reduced thickness of the unpolymerized layer, compared with Comparative Examples of similar constitutions.

In contrast, the dental compositions prepared in Comparative Examples failed to simultaneously achieve ambient light stability under high illuminance and photocurability, due to the differences in the ultraviolet absorbers employed.

In Comparative Examples 1-3, 1-6, 1-7, 2-3, 2-6, and 2-7, the ultraviolet absorbers had a solubility rating of "B", and satisfactory physical properties were considered unlikely. Accordingly, no other measurements were conducted.

### INDUSTRIAL APPLICABILITY

A dental composition according to the present invention can be suitably used in applications such as dental primers, dental bonding materials, and dental composite resins (such as dental self-adhesive composite resins) in the field of dentistry, with particular suitability as a dental bonding material.

## Claims

1. A dental composition comprising an ultraviolet absorber (A), a polymerizable monomer (B), and a photopolymerization initiator (C),
wherein the ultraviolet absorber (A) is a compound that has:
[I] a triazine skeleton;
[II] a solubility of 0.12 parts or more by mass relative to 100 parts by mass of 2-hydroxyethyl methacrylate; and
[III] a maximum absorption wavelength in a wavelength range of 310 nm or more and 400 nm or less as measured over a wavelength range of 250 nm to 500 nm for a solution prepared at a concentration of 3 × 10⁻⁵ mol/L by dissolving the ultraviolet absorber (A) in ethyl acetate serving as solvent, using a spectrophotometer with a quartz cell having an optical path length of 10 mm.

2. The dental composition according to claim 1, wherein the ultraviolet absorber (A) is a triazine-based ultraviolet absorber represented by the following general formula (1), 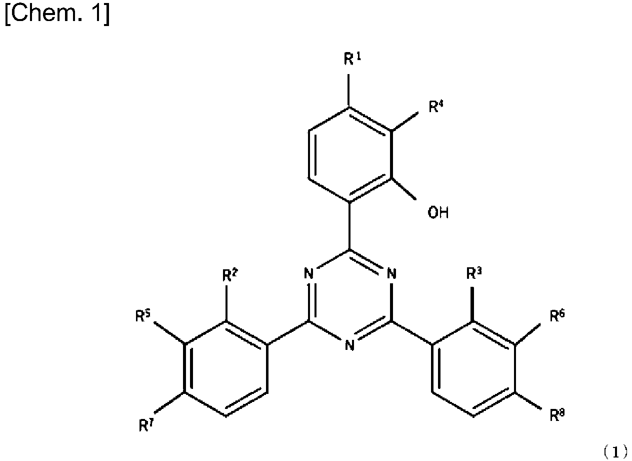 wherein R¹ represents an alkyl group or an alkoxy group, R² and R³ are the same or different from each other and each represent a hydrogen atom, a hydroxyl group, an alkyl group, an alkoxy group, or a halogen atom, R⁴, R⁵, and R⁶ are the same or different from each other and each represent a hydrogen atom or an alkyl group, and R⁷ and R⁸ are the same or different from each other and each represent a hydrogen atom, a hydroxyl group, an alkyl group, an alkoxy group, an aryl group, or a halogen atom, where the alkyl groups and alkoxy groups of R¹ to R⁸ may be optionally substituted.

3. The dental composition according to claim 1 or 2, wherein the content of the ultraviolet absorber (A) is 85 parts or more by mass in total 100 parts by mass of ultraviolet absorbers.

4. The dental composition according to claim 2, wherein R¹ in the ultraviolet absorber (A) is an alkyl group or alkoxy group having 1 to 15 carbon atoms.

5. The dental composition according to claim 4, wherein R¹ in the ultraviolet absorber (A) is a branched alkyl group or alkoxy group having 3 to 15 carbon atoms.

6. The dental composition according to claim 5, wherein R¹ in the ultraviolet absorber (A) is a 2-ethylhexyloxy group.

7. The dental composition according to claim 6, wherein the ultraviolet absorber (A) is bisethylhexyloxyphenol methoxyphenyl triazine and/or 2-[4,6-bis(biphenyl-4-yl)-1,3,5-triazin-2-yl]-5-[(2-ethylhexyl)oxy]phenol.

8. The dental composition according to claim 1 or 2, wherein the content of the ultraviolet absorber (A) is 0.01 parts or more by mass and 1 part or less by mass relative to total 100 parts by mass of the polymerizable monomer (B) contained in the dental composition.

9. The dental composition according to claim 1 or 2, wherein the mass ratio (A):(C) of the content of the ultraviolet absorber (A) and the content of the photopolymerization initiator (C) is 1:0.1 to 1:50.

10. The dental composition according to claim 1 or 2, wherein the photopolymerization initiator (C) is an α-diketone and/or a (bis)acylphosphine oxide.

11. The dental composition according to claim 10, wherein the content of the photopolymerization initiator (C) is 0.01 parts or more by mass and 5 parts or less by mass in 100 parts by mass of the dental composition.

12. The dental composition according to claim 1 or 2, wherein the dental composition is a one-pack type dental composition.
